# EUROPEAN PATENT SPECIFICATION

(11) **EP 1 439 870 B1**
(45) Date of publication and mention of the grant of the patent: **14.01.2009**
(21) Application number: 02803296.9
(22) Date of filing: 29.10.2002
(51) Int. Cl.: A61M 1/00

(54) **DEVICE FOR THE DELIVERY OF A SUBSTANCE**
VORRICHTUNG FÜR DIE ABGABE EINER SUBSTANZ
DISPOSITIF D'ADMINISTRATION D'UNE SUBSTANCE

(30) Priority: 29.10.2001 US 330713 P; 27.11.2001 US 333162 P
(43) Date of publication of application: 28.07.2004
(73) Proprietor: Becton, Dickinson and Company, Franklin Lakes, NJ 07417-1880 (US)
(72) Inventor: MIKSZTA, John, A., Durham, NC 27713 (US); PETTIS, Ronald, J., Cary, NC 27513 (US); ALARCON, Jason, Durham, NC 27703 (US); BRITTINGHAM, John, M., Wake Forest, NC 27587 (US); DEKKER, III, John, P., Cary, NC 27516 (US)
(74) Representative: Elend, Almut Susanne
(86) International application number: PCT/US2002/034504
(87) International publication number: WO 2003/051284

(56) References cited:
- EP-A- 1 086 719
- WO-A-02/32331
- WO-A-02/085446
- WO-A-03/026732
- FR-A- 2 498 443
- US-A- 4 109 655
- US-A- 4 597 764
- US-A- 5 999 847
- US-A- 6 077 229
- US-A- 6 132 755
- US-A1- 2002 053 756
- US-B1- 6 334 856

## Description

This application claims priority to U.S. Provisional patent application nos. 60/330,713, and 60/ 333,162 , filed October 29, 2001 and November 27, 2001 respectively.

### FIELD OF THE INVENTION

The present invention relates to a device for abrading the skin.

### BACKGROUND OF THE INVENTION

Delivery of substances to the body through the skin has typically been invasive, involving needles and syringes to facilitate intradermal (ID), intramuscular (IM) or subcutaneous (SC) injection. These methods are painful for the subject, require the skills of a trained practitioner and often produce bleeding. There have been efforts to overcome these disadvantages by use of devices which abrade the stratum corneum, the thin external layer of keratinized cells about 10-20 µm thick. The bioactive substance is delivered to the exposed viable epidermis.

This technique avoids the nerve net and places the bioactive substance in close proximity to blood vessels and lymphatics for absorption and delivery of the substance throughout the body.

For topical delivery of vaccines, the epidermis itself is a particularly desirable target as it is rich in antigen presenting cells. In comparison, the dermal layer below the epidermis contains fewer antigen presenting cells. Furthermore, the stratum corneum and epidermis do not contain nerves or blood vessels, so this method has the advantage of being essentially painless and blood-free while giving access to the skin layers capable of responding to the antigen.

The prior art reports a variety of devices and methods for disrupting the stratum corneum for the purpose of delivering substances to the body. For example, breach of the stratum corneum may be achieved by puncturing as taught in US Patent 5,679,647 to Carson, et al. This patent teaches that narrow diameter tines, such as those found on devices used for tuberculin skin tests and allergy tests, can be coated with polynucleotides or oligonucleotides and used for delivery of such materials into the skin. The method of using such devices involves puncturing the skin with the tines resulting in intracutaneous infection of the coated substance.

US Patent 5,003,987; US Patent 5,879,326; and US Patent 3,964,482 teach breaching the stratum corneum by cutting. EP 1 086 719 discloses a method and device for abrading skin prior to drug delivery. US 6,334,856 discloses a microncedle device for injecting fluid through the stratum corneum. WO02/08544 discloses a microprojection array for piercing or cutting the stratum corneum. WO02/3233 discloses a microstructure for exfoliating the skin. WO03/026732 discloses a switchable microneedle array.

### SUMMARY OF THE INVENTION

The present invention is directed to a device for abrading the skin, and particularly, the stratum corneum of the skin.

Substances to be delivered particularly include bioactive substances, including pharmaceutical agents, medicaments, vaccines and the like. Substances may be in solid or liquid form, depending on formulation and delivery method. They can be delivered *inter alia*, in the form of dry powders, gels, solutions, suspensions, and creams. Suitable formulations are familiar to those of skill in the art. Particularly preferred medicaments for delivery by the methods of the invention include vaccines, allergens and gene therapeutic agents.

One aspect of the invention is directed to a device for preparing a delivery site on the skin to enhance the delivery of a pharmaceutical agent through the stratum corneum of the skin to a sufficient depth where the pharmaceutical agent can be absorbed and utilized by the body.

Dermal tissue represents an attractive target site for delivery of vaccines and gene therapeutic agents. In the case of vaccines (both genetic and conventional), the skin is an attractive delivery site due to the high concentration of antigen presenting cells (APC) and APC precursors found within this tissue, especially the epidermal Langerhan's cells (LC). Several gene therapeutic agents are designed for the treatment of skin disorders, skin diseases and skin cancer. In such cases, direct delivery of the therapeutic agent to the affected skin tissue is desirable. In addition, skin cells are an attractive target for gene therapeutic agents, of which the encoded protein or proteins are active at sites distant from the skin. In such cases, skin cells (e.g., keratinocytes) can function as "bioreactors" producing a therapeutic protein which can be rapidly absorbed into the systemic circulation via the papillary dermis. In other cases, direct access of the vaccine or therapeutic agent to the systemic circulation is desirable for the treatment of disorders distant from the skin. In such cases, systemic distribution can be accomplished through the papillary dermis.

The present invention provides a microabrader device to abrade the skin in conjunction with the delivery of a bioactive substance, including but not limited to nucleic acids, amino acids, amino acid derivatives, peptides or polypeptides. It has been discovered that nucleic acids exhibit enhanced gene expression and produce an enhanced immune response to the expressed protein when they are delivered simultaneously with abrasion of the stratum corneum. Similarly, allergens delivered simultaneously with abrasion produce a more vigorous immune response than conventional allergen testing methods.

In one preferred embodiment, the present invention comprises a microabrader for delivering a substance into the skin having a base with an abrading facet, to which an abrading surface having an arrangement of microprotrusions that have at least one scraping edge is attached, mounted or integral with, and a handle attachment facet, to which a handle or other grasping device is attached, mounted, or integral with. By "abrading surface" is meant the surface that is presented to the skin during the process of abrasion, including microprotrusions, surface area between them and surrounding surface.

The microabrader device of the present invention is suitable for delivering a substance to the skin by movement of the microabrader device across an area of the skin to produce furrows of sufficient depth to allow the substance, which is administered prior to, simultaneously with, or following the abrasion of the skin, to be taken up by the predetermined skin layer. By means of the present microabrader device multiple passes of the device across the skin can result in progressively deeper furrows in the skin, thereby allowing delivery of a substance to a desired depth with in the skin.

### DESCRIPTION OF THE DRAWINGS

Figure 1A is an elevated view of the handle end of a preferred embodiment

Figure 1B is a side view of a preferred embodiment of a microabrader.

Figure 2A is a transparent perspective view of the microabrader device of Figures 1A and 1B.

Figure 2B is a cross sectional view of the microabrader device of Figure 1B.

Figure 3 is a side view of the abrading surface the microabrader device of Figures 1A; 1B, 2A, and 2B on the skin of a subject.

Figure 4 is a perspective view of the abrading surface in the embodiment of Figure 3.

Figure 4A is a cross sectional side view of the abrader surface.

Figure 5 is a bottom view of the abrader surface of the embodiment of Figure 3.

Figure 6 is a perspective view in partial cross section of abraded furrows of skin.

Figure 7 illustrates levels of reporter gene activity in skin obtained with the various nucleic acid delivery protocols tested in Example 1.

Figure 8 illustrates reporter gene activity in skin obtained by varying the number of abrasions as described in Example 2.

Figure 9 illustrates reporter gene activity in skin obtained by varying the formulation of the nucleic acid and the delivery protocol as described in Example 3.

Figure 10 illustrates the serum antibody response following delivery of plasmid DNA encoding Hepatitis B Surface Antigen (HBsAg) as described in Example 4.

Figure 11 illustrates mean luciferase activity (± SEM) in skin samples from rats treated with a reporter gene using Mantoux-style injection technique (Group 1A), plastic microneedle array of the invention (Group 2A), a tine device pressed against skin and scratched across an area of approximately 0.06 cm² (Group 3A), a tine device pressed against skin and moved across an area of approximately 1cm² (Group 4A), a tine device pressed against skin and removed (Group 5A), plasmid DNA directly applied in droplet form to the shaved skin (Group 6A).

Figure 12 shows skin reactions after application of histamine and abrading the skin of weaning pigs using a plastic microneedle array of the invention (Group 1 B), a tine device scratched once across an area of approximately 0.06 cm² (Group 2B), a tine device scratched multiple times to produce a scratched area of approximately 1 cm² (Group 3B), a time device pressed against the skin and removed (Group 4B). For each group, numbers 1-5 are replicates and "C" is a control to which histamine was applied without abrasion.

Figure 13 shows displays the relative area of tissue swelling for each group shown in Figure 12 after subtracting the swelling measurements observed from use of the device only without histamine.

Figures 14 and 15 compare Trans Epidermal Water Loss (TEWL) from skin following treatment with plastic and silicon microabraders.

Figure 16 illustrates reporter gene activity in skin following delivery of plasmid DNA encoding a reporter gene using plastic and silicon microabraders.

Figure 17 compares the serum antibody response following delivery of DNA plasmid encoding HBsAg using plastic and silicon microabraders.

Figure 18 illustrates the serum antibody response following administration of DNA plasmid encoding influenza hemagglutinin (HA), naked plasmid.

Figure 19 illustrates the serum antibody response following administration of DNA plasmid encoding influenza hemagglutinin (HA), plasmid plus adjuvant.

Figure 20 illustrates the serum antibody response following priming with naked plasmid DNA encoding influenza HA, followed by boosting with whole inactivated influenza virus.

Figure 21 illustrates the serum antibody response following priming with plasmid DNA encoding influenza HA plus adjuvant, followed by boosting with whole inactivated influenza virus.

Figure 22 illustrates the serum antibody response following administration of inactivated virus vaccine for rabies virus.

Figure 23 illustrates the serum antibody response following administration of HBsAg via the delivery protocols as described in Example 11a.

Figure 24 illustrates the T-cell proliferative response following administration of HbsAg.

Figure 25 illustrates the cellular immune response to a melanoma vaccine encoded by an adenoviral vector.

### DETAILED DESCRIPTION OF THE INVENTION

The present invention is directed to a device for abrading the stratum corneum to enhance the administering of a substance through the stratum corneum of the skin of a patient.

As used herein, the term "abrade" refers to removing at least a portion of the stratum corneum to increase the permeability of the skin without causing excessive skin irritation or compromising the skin's barrier to infectious agents. This is in contrast to "puncturing" which produces discrete holes through the stratum corneum with areas of undisrupted stratum corneum between the holes.

The microabrader of the invention is a device capable of abrading the skin to attain this result. In preferred embodiments, the device is capable of abrading the skin thereby penetrating the stratum corneum without piercing the stratum corneum. In one preferred embodiment, the microabrader also includes an effective amount of a substance to be delivered. This may be included, for example, in a reservoir that is an integral or detachable part of the microabrader, or may be coated on the delivery surface of the microabrader. By an "effective amount" of a substance is intended to mean an amount that will elicit a desired response in a subject, including, but not limited to, an immunostimulatory or immunomodulatory response in the case of an allergen or vaccine, or another therapeutic or diagnostic response.

As used herein, "penetrating" refers to entering the stratum corneum without passing completely through the stratum corneum and entering into the adjacent layers. This is not to say that that the stratum corneum can not be completely penetrated to reveal the interface of the underlying layer of the skin. Piercing, on the other hand, refers to passing through the stratum corneum completely and entering into the adjacent layers below the stratum corneum.

The microabrader device of the invention is believed to have a unique immunological advantage in the delivery of vaccines with the potential of increasing the vaccine's clinical value. The penetration of the multiple microprotrusions into the stratum corneum is suggested as having an adjuvant-like stimulatory effect. The "penetration" response from the multiple microprotrusion is believed more than a simple acute inflammatory response. These "penetration" effects can cause damage to a variety of cells and cellular architecture, causing the appearance of polymorphonuclear neutrophils (PMN) and macrophages as well as the release of IL1, tumor necrosis factor (TNF) and other agents, which can lead to a number of other immunological responses. The soluble stimulatory factors influence the proliferation of lymphocytes and are central to the immune response to vaccines. In addition, these factors influence the migration and activation of resident antigen presenting cells including Langerhan's cells and dendritic cells. The microabrader of the present invention is valuable in promoting significant immune response to a vaccine in the abraded area. The small grooves and furrows created by the microprotrusion array over the abraded area are believed to increase the availability of the vaccine antigen for interaction with antigen-presenting cells compared to a vaccine applied topically in the absence of abrasion or administered using standard needles.

The microprotrusion array of the invention is believed to magnify several-fold the trivial or inconsequential immune stimulatory impact of a single needlestick. The microabrader facilitates and enhances vaccine immunogenicity by an adjuvant-like immune stimulation.

The primary barrier properties of the skin including the resistance to delivery of drugs, vaccines and gene therapeutic agents reside in the outermost layer of the epidermis, referred to as the stratum corneum. The inner layers of the epidermis generally include three layers, commonly identified as the stratum granulosum, the stratum malpighii, and the stratum germinativum. Once a drug or other substance appears below the stratum corneum, there is essentially no resistance to diffusion into subsequent layers of the skin and eventual uptake by cells or absorption by the body through the bloodstream or lymphatic drainage.

Helping a substance to pass through the stratum corneum can be an effective method for facilitating absorption of some substances, and particularly some vaccines, by the body. The present invention is primarily directed to a device and method for facilitating delivery of a substance, and particularly a bioactive substance or pharmaceutical agent, into or through the stratum corneum for more rapid absorption of larger quantities of the bioactive substance or pharmaceutical agent by the patient.

Preferably, the device of the invention penetrates, but does not pierce, the stratum corneum. The substance to be administered using the methods of this invention may be applied to the skin prior to abrading, simultaneous with abrading, or post-abrading. According to one embodiment of the methods of the invention, however, certain or specific bioactive substances, including nucleic acids , allergens and live viral vaccines are applied to the skin prior to or simultaneously with abrasion rather than being applied to previously abraded skin. That is, delivery of certain substances, such as nucleic acids, allergens and live viral vaccines are improved when such substances are abraded into the skin rather than being passively applied to skin which has been previously abraded. In another embodiment of the method of the invention, however, certain or specific bioactive substances, including virus-like particles and subunit proteins, are improved when such substances are applied to pre-abraded skin. In other embodiments of the method of the invention, however, certain or specific bioactive substances, including whole inactivated or killed viruses, display similar efficacy whether applied to skin following abrasion or simultaneously with abrasion.

The substance may be delivered into the skin in any pharmaceutically acceptable form. In one embodiment the substance is applied to the skin and an abrading device is then moved or rubbed reciprocally over the skin and the substance. It is preferred that the minimum amount of abrasion to produce the desired result be used. Determination of the appropriate amount of abrasion for a selected substance is within the ordinary skill in the art. In another embodiment the substance may be applied in dry form to the abrading surface of the delivery device prior to application. In this embodiment, a reconstituting liquid is applied to the skin at the delivery site and the substance-coated abrading device is applied to the skin at the site of the reconstituting liquid. It is then moved or rubbed reciprocally over the skin so that the substance becomes dissolved in the reconstituting liquid on the surface of the skin and is delivered simultaneously with abrasion. Alternatively, a reconstituting liquid may be contained in the abrading device and released to dissolve the substance as the device is applied to the skin for abrasion. It has been found that certain substances, such as nucleic acid preparations, may also be coated on the abrading device in the form of a gel.

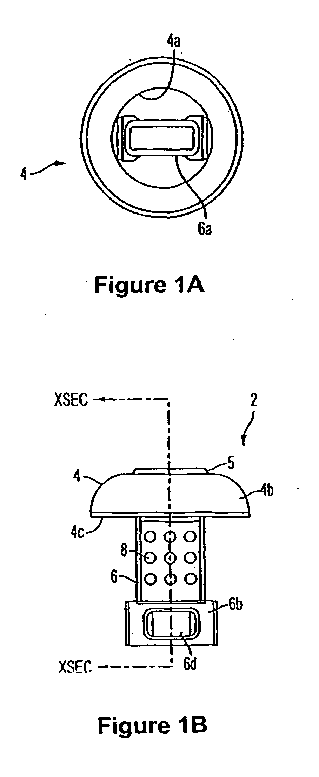

A method for delivering a substance into the skin of a patient, which is not part of the invention, includes the steps of coating a patient's outer skin layer or a microabrader 2 , see Figure 1B with a medicament or other substance and moving microabrader 2 across the patient's skin to provide abrasions leaving furrows sufficient to permit entry of the substance into the patient's viable epidermis. Due to the structural design of microabrader 2, the leading edge of microabrader 2 first stretches the patient's skin and then the top surface of microabrader 2 abrades the outer protective skin layer opening the stratum corneum thereby permitting medicament or other substance to enter the patient. After the initial abrasion of the outer protective skin layer, the trailing and leading edges of microabrader 2 can rub the surface of the abraded area working the medicament or substance into the abraded skin area thereby improving its medicinal effect.

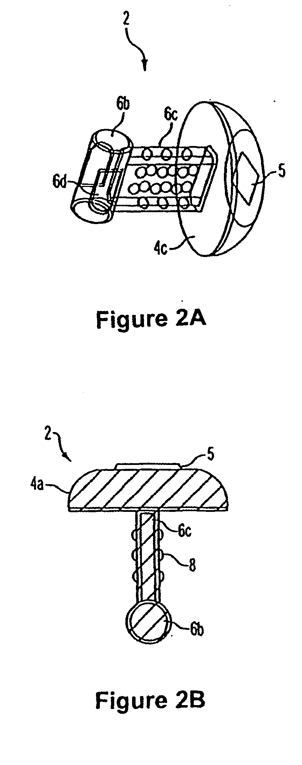

As shown in Figures 1B, 2A and 2B, microabrader 2 includes base 4 onto which an abrading surface 5 can be mounted. Alternatively, the abrading surface may be integral with the base and fabricated as a single two-component part. Preferably, base 4 is a solid molded piece. In one embodiment, base 4 is configured with a mushroom-like crown 4b that curves upward and is truncated at the top. The top of base 4 is generally flat with abrading surface 5 being mounted thereon or integral therewith. Alternatively, the truncated top may have a recess for receiving abrading surface 5. In all embodiments, abrading surface 5 includes a platform with an array of microprotrusions that extends above the truncated top. In another embodiment of the microabrader, the handle, base and abrading surface may be integral with one another and fabricated as a single three-component device.

Microabrader 2 is applied to a subject by moving microabrader 2 across the subject's skin with enough pressure to enable abrading surface 5 to open the outer protective skin or stratum corneum of the subject. The inward pressure applied to the base causes microabrader 2 to be pressed into the subject's skin. Accordingly, it is preferable that the height of the sloping mushroom-like crown 4b be sufficient to prevent the applied substance from flowing over and onto the facet 4c when microabrader 2 is being used. As will be described below, abrading surface 5 comprises an array of microprotrusions.

A handle 6 is attached to base 4 or may be integral with base 4. As shown in Fig. 2A, an upper end 6a of the handle may be either snap fit or friction fit between the inner circumferential sidewall 4a of base 4. Alternatively, as shown in Figures 1A and 2A, handle 6 may be glued (e.g., with epoxy) to the underside 4c of base 4. Alternatively, the handle and base may be fabricated (e.g., injection-molded) together as a single two-component part. The handle may be of a diameter that is less than the diameter of the base or may be of a similar diameter as the base. Underside 4c of base 4 may be flush with mushroom-like crown 4b or extend beyond the mushroom-like crown. The lower end 6b of handle 6 may be wider than the shaft 6c of handle 6 or may be of a similar diameter as shaft. Lower end 6b may include an impression 6d that serves as a thumb rest for a person administering the substance and moving microabrader 2. In addition, protrusions 8 are formed on the outside of handle 6 to assist a user in firmly gripping handle 6 when moving the same against or across a patient's skin.

As shown in the cross-section of Figure 1B in Figure 2B, lower end 6b may be cylindrical. Microabrader 2 may be made of a transparent material, as shown in Figure 2A. Impressions 6d are disposed on both sides of the cylindrical lower end 6b to assist a person using microabrader 2 to grip the same. That is, the movement of microabrader 2 can be provided by hand or fingers. The handle 6, as well as the base 4, of the microabrader is preferably molded out of plastic or the like material. The microabrader 2 is preferably inexpensively manufactured so that the entire microabrader and abrading surface can be disposed after its use on one patient.

Abrading surface 5 is designed so that when microabrader 2 is moved across a patient's skin, the resultant abrasions penetrate the stratum corneum. Abrading surface 5 may be coated with a medicament or substance desired to be delivered to the patient's viable epidermis.

In order to achieve the desired abrasions, the microabrader 2 should be moved across a patient's skin at least once. The patient's skin may be abraded in alternating directions. The structural design of the microabrader according to the invention enables the medicament or substance to be absorbed more effectively thereby allowing less of the medicament or substance to be applied to a patient's skin or coating abrading surface 5.

Abrading surface 5 may be coated with a substance desired to be delivered to the patient. In one embodiment, the substance may be a powder disposed on abrading surface 5. In another embodiment, the substance to be delivered may be applied directly to the patient's skin prior to the application and movement of microabrader 2 on the patient's skin.

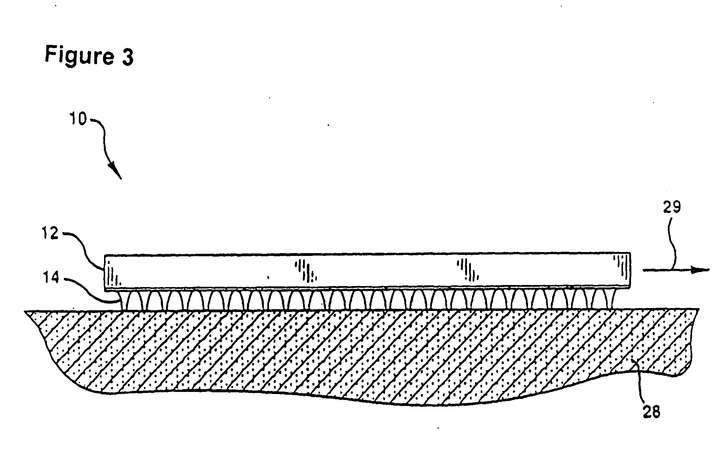

Referring to Figure 3, the microabrader device 10 of the invention includes a substantially planar body or abrading surface support 12 having a plurality of microprotrusions 14 extending from the bottom surface of the support. The support generally has a thickness sufficient to allow attachment of the surface to the base of the microabrader device thereby allowing the device to be handled easily as shown in Figure 1B, 2A and 2B. Alternatively, a differing handle or gripping device can be attached to or be integral with the top surface of the abrading surface support 12. The dimensions of the abrading surface support 12 can vary depending on the length of the microprotrusions, the number of microprotrusions in a given area and the amount of the substance to be administered to the patient. Typically, the abrading surface support 12 has a surface area of about 1 to 4 cm². In preferred embodiments, the abrading surface support 12 has a surface area of about 1 cm².

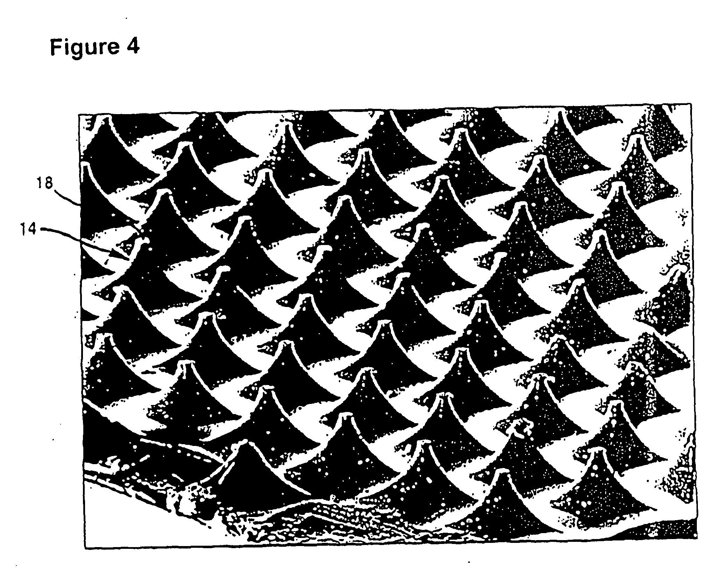

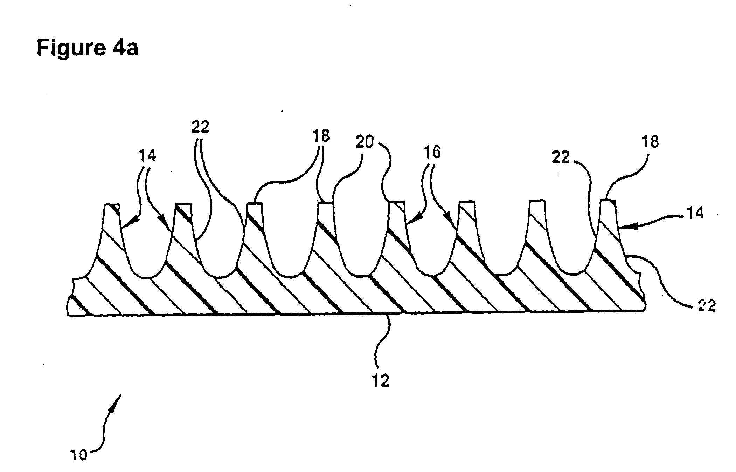

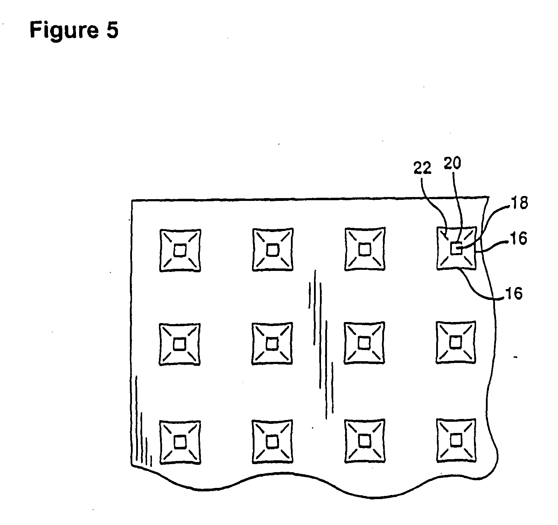

As shown in Figures 3, 4, 4A and 5, the microprotrusions 14 project from the surface of the abrading surface support 12 and are substantially perpendicular to the plane of the abrading surface support 12. The microprotrusions in the illustrated embodiment are arranged in a plurality of rows and columns and are preferably spaced apart a uniform distance. The microprotrusions 14 have a generally pyramid shape with sides 16 extending to a tip 18. The sides 16 as shown have a generally concave profile when viewed in cross-section and form a curved surface extending from the abrading surface support 12 to the tip 18. In the embodiment illustrated, the microprotrusions are formed by four sides 16 of substantially equal shape and dimension. As shown in Figures 4A and 5, each of the sides 16 of the microprotrusions 14 have opposite side edges contiguous with an adjacent side and form a scraping edge 22 extending outward from the abrading surface support 12. The scraping edges 22 define a generally triangular or trapezoidal scraping surface corresponding to the shape of the side 16. In further embodiments, the microprotrusions 14 can be formed with fewer or more sides.

The microprotrusions 14 preferably terminate at blunt tips 18. Generally, the tip 18 is substantially flat and parallel to the support 14. When the tips are flat, the total length of the microprotrusions do not penetrate the skin; thus, the length of the microprotrusions is greater than the total depth to which said microprotrusions penetrate said skin. The tip 18 preferably forms a well defined, sharp edge 20 where it meets the sides 16. The edge 20 extends substantially parallel to the abrading surface support 12 and defines a further scraping edge. In further embodiments, the edge 20 can be slightly rounded to form a smooth transition from the sides 16 to the tip 18. Preferably, the microprotrusions are frustoconical or frustopyramidal in shape.

The microabrader device 10 and the microprotrusions can be made from a plastic material that is non-reactive with the substance being administered. A non-inclusive list of suitable plastic materials include, for example, polyethylene, polypropylene, polyamides, polystyrenes, polyesters, and polycarbonates as known in the art. Alternatively, the microprotrusions can be made from a metal such as stainless steel, tungsten steel, alloys of nickel, molybdenum, chromium, cobalt, titanium, and alloys thereof, or other materials such as silicon, ceramics and glass polymers. Metal microprotrusions can be manufactured using various techniques similar to photolithographic etching of a silicon wafer or micromachining using a diamond tipped mill as known in the art. The microprotrusions can also be manufactured by photolithographic etching of a silicon wafer using standard techniques as are known in the art. They can also be manufactured in plastic via an injection molding process, as described for example in U.S. application no. 10/193,317, filed July 12, 2002, which is hereby incorporated by reference.

The length and thickness of the microprotrusions are selected based on the particular substance being administered and the thickness of the stratum corneum in the location where the device is to be applied. Preferably, the microprotrusions penetrate the stratum corneum substantially without piercing or passing through the stratum corneum. The microprotrusions can have a length up to about 500 microns. Suitable microprotrusions have a length of about 50 to 500 microns. Preferably, the microprotrusions have a length of about 50 to about 300 microns, and more preferably in the range of about 150 to 250 microns, with 180 to 220 microns most preferred. The microprotrusions in the illustrated embodiment have a generally pyramidal shape and are perpendicular to the plane of the device. These shapes have particular advantages in insuring that abrasion occurs to the desired depth. In preferred embodiments, the microprotrusions are solid members. In alternative embodiments, the microprotrusions can be hollow.

As shown in Figure 2 and 5, the microprotrusions are preferably spaced apart uniformly in rows and columns to form an array for contacting the skin and penetrating the stratum corneum during abrasion. The spacing between the microprotrusions can be varied depending on the substance being administered either on the surface of the skin or within the tissue of the skin. Typically, the rows of microprotrusions are spaced to provide a density of about 2 to about 10 per millimeter (mm). Generally, the rows or columns are spaced apart a distance substantially equal to the spacing of the microprotrusions in the array to provide a microprotrusion density of about 4 to about 100 microprotrusions per mm². In another embodiment, the microprotrusions may be arranged in a circular pattern. In yet another embodiment, the microprotrusions may be arranged in a random pattern. When arranged in columns and rows, the distance between the centers of the microprotrusions is preferably at least twice the length of the microprotrusions. In one preferred embodiment, the distance between the centers of the microprotrusions is twice the length of the microprotrusions ±10 microns. Wider spacings are also included, up to 3, 4, 5 and greater multiples of the length of the micoprotrusions. In addition, as noted above, the configuration of the microprotrusions can be such, that the height to the microprotrusions can be greater than the depth into the skin those protrusions will penetrate.

The flat upper surface of the frustoconical or frustopyramidal microprotrusions is generally 10 to 100, preferably 30-70, and most preferably 35-50 microns in width.

The method of preparing a delivery site on the skin, which is not part of the invention places the microabrader against the skin 28 of the patient in the desired location. The microabrader is gently pressed against the skin and then moved over or across the skin. The length of the stroke of the microabrader can vary depending on the desired size of the delivery site, defined by the delivery area desired. The dimensions of the delivery site are selected to accomplish the intended result and can vary depending on the substance, and the form of the substance, being delivered. For example, the delivery site can cover a large area for treating a rash or a skin disease. Generally, the microabrader is moved about 2 to 15 centimeters (cm). In some embodiments of the invention, the microabrader is moved to produce an abraded site having a surface area of about 4 cm² to about 300 cm².

The microabrader is then lifted from the skin to expose the abraded area and a suitable delivery device, patch or topical formulation may be applied to the abraded area. Alternatively, the substance to be administered may be applied to the surface of the skin either before, or simultaneously with abrasion.

The extent of the abrasion of the stratum corneum is dependent on the pressure applied during movement and the number of repetitions with the microabrader. In one embodiment, the microabrader is lifted from the skin after making the first pass and placed back onto the starting position in substantially the same place and position. The microabrader is then moved a second time in the same direction and for the same distance. In another embodiment, the microabrader is moved repetitively across the same site in alternating direction without being lifted from the skin after making the first pass. Generally, two or more passes are made with the microabrader.

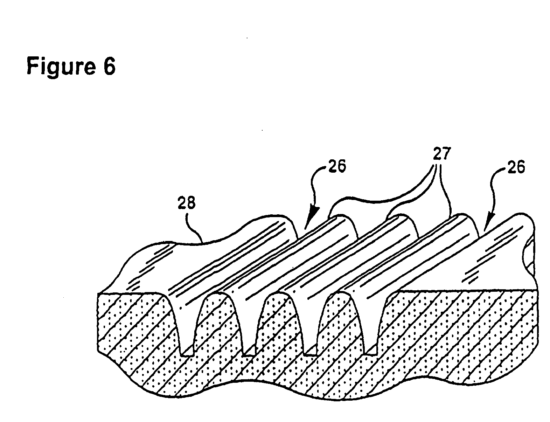

In further embodiments, the microabrader can be swiped back and forth, in the same direction only, in a grid-like pattern, a circular pattern, or in some other pattern for a time sufficient to abrade the stratum corneum a suitable depth to enhance the delivery of the desired substance. The linear movement of the microabrader across the skin 28 in one direction removes some of the tissue to form grooves 26, separated by peaks 27 in the skin 28 corresponding to substantially each row of microprotrusions as shown in Figure 6. The edges 20, 22 and the blunt tip 18 of the microprotrusions provide a scraping or abrading action to remove a portion of the stratum corneum to form a groove or furrow in the skin rather than a simple cutting action. The edges 20 of the blunt tips 18 of the microprotrusions 14 scrape and remove some of the tissue at the bottom of the grooves 26 and allows them to remain open, thereby allowing the substance to enter the grooves for absorption by the body. Preferably, the microprotrusions 14 are of sufficient length to penetrate the stratum corneum and to form grooves 26 having sufficient depth to allow absorption of the substance applied to the abraded area without inducing pain or unnecessary discomfort to the patient. Preferably, the grooves 26 do not pierce but can extend through the stratum corneum. The edges 22 of the pyramid shaped microprotrusions 14 form scraping edges that extend from the abrading surface support 12 to the tip 18. The edges 22 adjacent the abrading surface support 12 form scraping surfaces between the microprotrusions which scrape and abrade the peaks 27 formed by the skin between the grooves 26. The peaks 27 formed between the grooves generally are abraded slightly.

In further embodiments, the microabrader can include a dried or lyophilized pharmaceutical agent on the support or on or between the microprotrusions. The dried pharmaceutical agent can be applied as a coating on the microprotrusions or in the valleys between the microprotrusions. During abrasion of the skin, the pharmaceutical agent is transferred to the abraded area of the skin. The microabrader can remain in place on the abraded delivery site for a sufficient time to allow the pharmaceutical agent to pass through the abraded delivery site into the epidermis. The microabrader can be attached to the skin by an adhesive tape or patch covering the microabrader. Preferably, the microabrader is attached to the abraded delivery site as prepared by the above method where the pharmaceutical agent is passively delivered without the use of a diluent or solvent.

In further embodiments, a suitable solvent or diluent such as distilled water or saline solution can be injected through an opening in the support to solubilize and reconstitute the pharmaceutical agent while the microabrader is attached to the delivery site. The solvent or diluent can be injected from a syringe or other container, or be contained in a reservoir that is an integral part of the microabrader device.

Preferably, the microprotrusions are uniformly spaced apart to form an array and have a substantially uniform length and width. In a further embodiment, the microprotrusions have varying lengths to penetrate the skin at different depths. Varying the length of the microprotrusions allows the substance to be deposited at different depths in the skin and can increase the effectiveness of the delivery.

If the abrading device does not include a reservoir for containment and discharge of fluids from the device, the substance-containing liquid or the reconstituting liquid must be separately applied to the skin prior to or after abrading, for example from a separate dispenser or pump. However, reservoirs may be an integral part of the abrading device. Preferably the reservoir is in fluid communication with the abrading surface of the device or skin, for example via channels through the needles or protrusions, or via channels which exit the reservoir between such needles or protrusions, or via porous materials, or adjacent to the abrading surface. In this embodiment, the substance or reconstituting liquid is contained in the reservoir of the abrading device and is dispensed to the skin surface prior to abrasion, simultaneously with abrasion, or after abrasion. The abrading device may also include means for controlling the rate of delivery of the substance or reconstituting liquid, or for controlling the amount of substance or reconstituting liquid delivered. As an alternative, a patch, either dry or pre-moistened, may be applied to the site subsequent to abrasion to facilitate reconstitution, or enhance introduction or uptake of substances into the skin. In another embodiment, the patch may contain the medicament and may be applied to skin that was pre-treated with a microabrader device.

Nucleic acids for use in the methods of the invention may be RNA or DNA. A nucleic acid may be in any physical form suitable for topical administration and for uptake and expression by cells. It may be contained in a viral vector, liposome, particle, microparticle, nanoparticle, or other suitable formulation as is known in the art, or it may be delivered as a free polynucleotide such as a plasmid as is known in the art. The nucleic acid will typically be formulated in a pharmaceutically acceptable formulation such as a fluid or gel which is compatible with the nucleic acid. Pharmaceutically acceptable formulations for use in the invention, including formulations for vaccines and allergen compositions, are also well known in the art.

It has been found that minimal abrasion (as little as one pass over the skin) is sufficient to produce an improvement in nucleic acid delivery to skin cells. The amount of nucleic acid delivery and expression continues to increase with increasing, numbers of abrasive passes over the skin. Six abrasive passes or more gave the maximum improvement in nucleic acid delivery in experimental animal studies. Although all abrasive passes over the skin may be in the same direction, it is preferred that the direction be altered during abrasion. The most commonly used protocol for delivery of nucleic acid vaccines today is IM injection, usually with additional response enhancers when the dose is low. Determination of the appropriate dose of nucleic acid vaccine to be delivered using the methods of the invention is within the ordinary skill in the art. However, it is an advantage of the inventive methods that delivery of nucleic acid vaccines is more efficient than IM delivery even without response enhancers, as evidenced by levels of gene expression and stimulation of an immune response.

Amino acids, amino acid derivatives, peptides and polypeptides, particularly allergens, may also be delivered topically according to the device and methods of the invention. Allergens are conventionally delivered into the skin by intracutaneous puncture using devices similar to the tuberculin tine test. However, it has been unexpectedly found that an enhanced allergenic response can be obtained by simultaneous abrasion and delivery. This produces a more sensitive test and has the advantage that a minor or imperceptible response to the conventional allergen test may be more easily detected using the methods of the invention. Thus, the devices and methods of the invention result in better performance & less skin irritation and erthyma than methods using tine-based devices previously known in the art. Other suitable abraders for delivery of vaccines as well as other medicaments include those disclosed in U.S. application no. 09/405,488, filed September 24, 1999. It will be appreciated that the size and shape of the surface area of the abrader, and the shape and pattern of the needles or protrusions can vary according to the particular vaccine or other agent to be delivered and other factors such as ease of application and efficacy, as will be appreciated by those of skill in the art.

Typically, to administer vaccine or other medicament using the present invention, a practitioner will remove the appropriate volume from a vial sealed with a septa using a syringe, and apply the vaccine or medicament to the skin either before or following abrasion using the microabrader. This procedure will at a minimum result in the use of both a syringe needle and a microabrader for each administration procedure, and require time and attention for dosage measurement. Thus, it would be desirable to provide for a kit including the microabrader device either in combination with or adapted to integrate therewith, the substance to be delivered.

Kits and the like comprising the instrument of administration and the therapeutic composition are well known in the art. However, the application of minimally invasive, microabrader devices for the delivery of drugs and vaccines clearly present an immediate need for coupling the device with the formulation to provide safe, efficacious, economic and consistent means for administering formulations for enabling immunogenic or other therapeutic responses.

The kit provided by the invention comprises at least one microabrader delivery device having an abrading surface, wherein said abrading surface comprises microprotrusions projecting from and arranged in patterns and wherein said microprotrusions comprise at least one scraping edge. The microabrader delivery device contained in the kit may be fully integrated, i.e. include a facet adapted to receive or integral with said abrading surface, a handle attachment facet, and a handle that is integral with or detachable from said base. A reservoir containing a vaccine or other medicament, and means to effect delivery may also be integrated into the delivery device. Alternatively, the kit may contain only parts of the microabrader that may be considered disposable (for example, the abrading surface and medicament doses), with reusable items such as the handle and facet being separately supplied. Such kits may, for example, comprise multiple attachable abrading surfaces and multiple vaccine dosages suitable for mass inoculations, with handles and facets being supplied separately (optionally in smaller numbers). Alternatively, the kit may contain one or more complete "one use" microabrader devices that include the abrading surface, facet, handle in "use and dispose" form. In one preferred embodiment, the kit also contains means for containing, measuring, and/or delivering a dosage of a vaccine or other medicament. In a particularly preferred embodiment, the kit also contains an effective dosage of a vaccine or other medicament, optionally contained in a reservoir that is an integral part of, or is capable of being functionally attached to, the delivery device. Alternatively, the vaccine or other medicament may be supplied in a patch that is packaged in a kit also comprising an abrasion device. In this embodiment, the abrasion device is first used to treat the skin, after which the patch is applied to the treated skin site.

In one particularly preferred embodiment, the kit of the invention comprises a microabrader coated with an effective amount of the medicament or vaccine to be administered. By an "effective amount" or "effective dosage" of a substance is intended to mean an amount that will elicit a desired response in a subject, including, but not limited to, an immunostimulatory response in the case of an allergen or vaccine, or other therapeutic or diagnostic response.

To use a kit as envisioned by the instant invention the practitioner would break a hermetic seal to provide access to the microabrader device and optionally, the vaccine or immunogenic or therapeutic composition. The composition may be preloaded into a reservoir contained in the microabrader device or a separate application device in any suitable form, including but not limited to gel, paste, oil, emulsion, particle, nanoparticle, microparticle, suspension or liquid, or coated on the microabrader device in a suitable dosage. The composition may be separately packaged within the kit package, for example, in a reservoir, vial, tube, blister, pouch, patch or the like. One or more of the constituents of the formulation may be lyophilized, freeze-dried, spray freeze-dried, or in any other reconstitutable form. Various reconstitution media, cleansing or disinfective agents, or topical steriliants (alcohol wipes, iodine) can further be provided if desired. The practitioner would then apply the formulation to the skin of the patient either before or following the abrasion step, or in the case of a preloaded or precoated microabrader device, carry out the abrasion step without separate application of the medicament.

### EXAMPLE 1

### DELIVERY OF PLASMID DNA ENCODING A REPORTER GENE USING A MICROABRADER DEVICE

Plasmid DNA (35 µg) encoding firefly luciferase was administered to anesthetized BALB/c mice by IM injection or ID injection with a standard 30 g needle and 1 cc syringe, or was administered topically using a microabrader device comprising an abrading surface consisting of 200mm length silicon frustoconical microprojections, as shown in Figure 4. The abrading surface was fitted onto a microabrader device, as depicted in Figures 1 and 2.

Two protocols were used for DNA administration using the microabrader device:
1) Simultaneous abrasion and delivery (ABRdel): Mice were shaved on the caudal dorsum using electric clippers, followed by a No. 10 scalpel blade to remove remaining hair. The DNA solution was then applied to a 1 cm² site on the skin surface and the abrading surface of the microabrader device was placed in contact with this solution and then the microabrader device was moved laterally in alternating direction six times across the skin surface (three passes in each direction). The DNA solution was left to air dry and the skin site was left uncovered until skin biopsies were recovered.
2) Pre-abrasion (preABR): After shaving as described above, a 1 cm² site was pre- abraded by lateral movement of the microabrader device across the skin surface six times with alternating direction (three passes in each of two directions). The DNA solution was then spread over the abraded skin surface and left to air dry as above.

As a control for possible DNA delivery through hair follicles or nicks resulting from the shaving process, animals were shaved as above but were not abraded with the microabrader device (noABR). The DNA solution was applied topically to the 1 cm² shaved skin site and left to air dry.

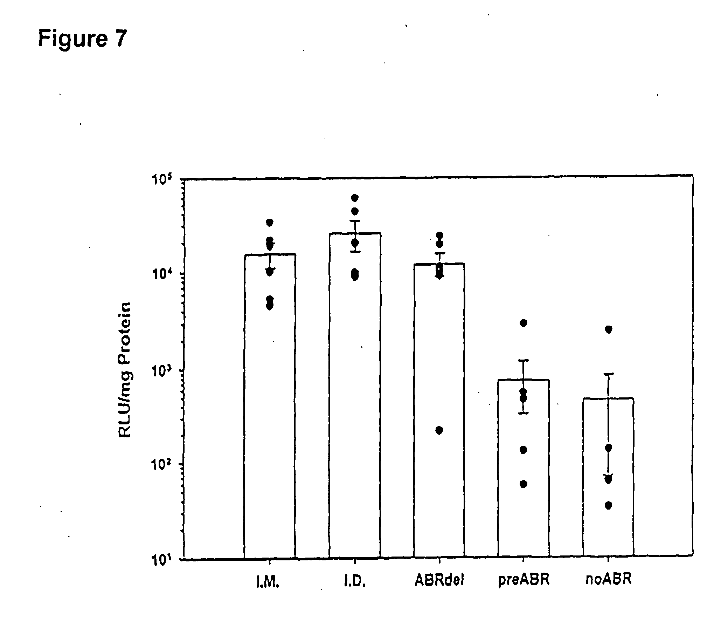

In all groups, tissue samples were collected 24 hr. after DNA administration. Tissue homogenates were analyzed for luciferase activity using a luminescence assay. All samples were normalized for total protein content, as determined by a standard BCA protein assay. Data were expressed as Relative Light Units (RLU) per mg of total protein and results are shown in Figure 7 . Each symbol represents the response of a single mouse. Cumulative data from two separate experiments are shown (n=6 for each group). The levels of luciferase reporter gene activity attained following ABRdel were similar in magnitude to needle-based IM and ID injections and significantly greater (p=0.02) than for topical delivery onto pre-abraded or unabraded skin.

### EXAMPLE 2

### CORRELATION OF DELIVERY WITH NUMBER OF ABRASIVE PASSES

Luciferase plasmid DNA (35 µg) was administered by ABRdel as described in Example 1, but the number of lateral passes of the microabrader device across the skin surface was varied (12, 10, 6, 4 and 2 times). In addition, after placing the DNA solution on the surface of shaved but unabraded skin, the abrading surface of the microabrader device was repetitively pressed against the skin (six times) to simulate puncture-mediated delivery. Topical application of the DNA solution in the absence of abrasion (noABR) was included as a control for possible DNA delivery through hair follicles or nicks. Skin biopsies (1 cm²) were collected 24 hr. after application and were assayed for luciferase activity as described in Example 1.

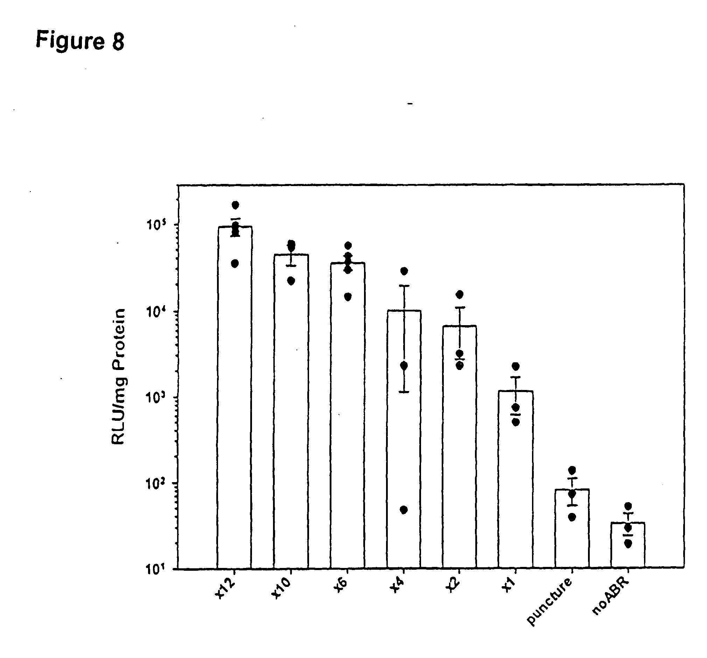

The results are illustrated in Figure 8. Each symbol represents the response of a single mouse, and n=3 for all groups except for "x12" and "x6" in which n=5. Increasing levels of gene expression were attained with increasing numbers of passes of the microabrader device across the skin surface. Mean levels of expression ranged from greater than 1,000- to 2,800-fold above noABR controls in groups treated by six or more abrasions. Mean responses following 4, 2, or 1 pass of the microabrader device across the surface of the skin were about 300-, 200- and 30-fold above background, respectively. Mean levels of expression in the "puncture" group were only 2-fold above background and were not significantly different from no ABR controls.

These data demonstrate that the abrasion process is a critical component of topical delivery of DNA into the skin. Increased levels of gene expression were attained by increasing the number of abrasive passes of the microabrader device, although gene expression was observed after even a single pass. In addition, laterally rubbing or abrading the skin significantly increased nucleic acid delivery and gene expression as compared to repetitively pressing the microabrader device against the skin without lateral abrasion.

### EXAMPLE 3

### FORMULATION OF PLASMID DNA

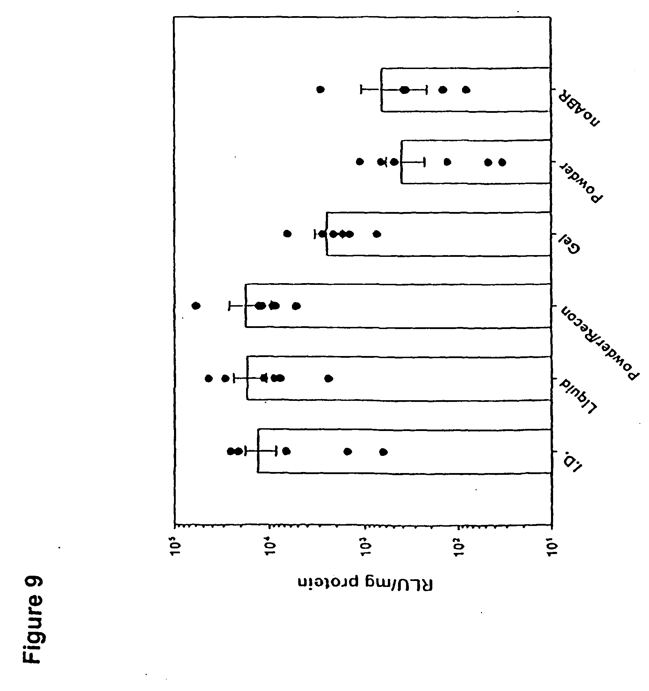

Luciferase plasmid (35 µg) was administered as a liquid formulation by ID injection or by simultaneous abrasion and delivery ("ABRdel liquid") with six passes of the microabrader device across the skin surface as described in Example 1. In addition, the DNA was lyophilized to a powder and coated onto the surface of the abrading surface of the microabrader device and administered by simultaneous abrasion and delivery either directly as a powder ("ABRdel powder") or upon reconstitution in PBS buffer at the time of application ("ABRdel powder/recon"). Reconstitution was accomplished by placing the powder-coated abrading surface in direct contact with a droplet of PBS on the surface of . the skin, followed by simultaneous abrasion and delivery. Abrading surfaces of microabrader devices were also coated with DNA dissolved in 0.5% agarose gel and administered by simultaneous abrasion and delivery as described above ("ABRdel gel"). Topical application of the liquid formulation in the absence of abrasion (noABR) was included as a control. Skin biopsies (1 cm²) were collected 24 hr. after application and were assayed as described in Example 1. The results are shown in Figure 9. Each symbol represents the response of a single mouse. Cumulative data from two separate experiments are shown, where n=6 for each group. Similar levels of luciferase expression in the skin (about 20-30 fold above noABR) were observed for the ID injection, ABRdel liquid and ABRdel powder/recon groups. Although neither direct delivery of gel or powder-coated DNA without reconstitution resulted in gene expression statistically above the noABR control, responses following direct gel-based delivery were about 2-10 fold higher than the mean control response. These results demonstrate that reconstitution of a dry form of the vaccine at the time of simultaneous abrasion and delivery produces results comparable to simultaneous abrasion and delivery of a liquid formulation. This has advantages for commercial application of the methods, as an abrader device with a liquid-filled reservoir could be pre-coated with the vaccine powder for reconstitution of the vaccine as it is applied by abrasion.

### EXAMPLE 4

### ANTIBODY RESPONSE FOLLOWING DELIVERY OF DNA VACCINE FOR HEPATITIS B VIA MICROABRADER DEVICE

Plasmid DNA encoding the Hepatitis B surface antigen (HBsAg) was administered to anaesthetized BALB/c mice by IM or ID injection with a standard 30 g needle and 1 cc syringe, or was administered using a microabrader device as described in Example 1 according to the ABRdel protocol of Example 1. Mice were given a total of three immunizations of 100 µg per dose. Serum samples were analyzed by ELISA for antibodies to HBsAg (total Ig) 2-3 weeks following each immunization. DNA was applied topically to shaven but unabraded (noABR) skin as control for possible delivery through nicks or hair follicles. Data represent an anti-HBsAg titer, defined as the highest dilution of a serum sample yielding absorbance values at least three times above background (serum obtained from naive, unimmunized mice).

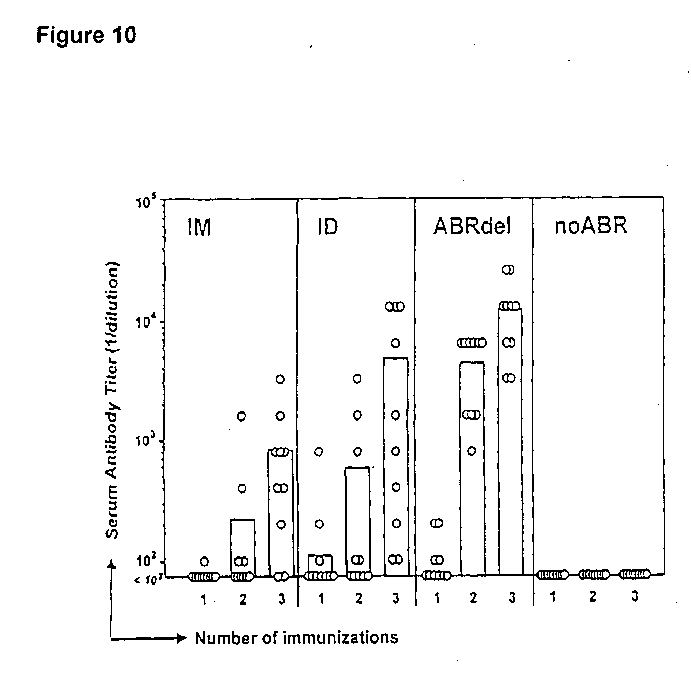

A total of ten mice per group were analyzed. Mean titers are represented as bars in Figure 10, with the responses of individual mice indicated as open symbols. The results indicate that administration of DNA vaccines using the microabrader device according to the ABRdel protocol induces strong serum antibody responses in vivo. The magnitude of such responses were significantly greater (p<0.05 after immunizations 2 and 3) than those induced via either IM (the current standard for DNA-based vaccine delivery) and ID injections. In addition, the responses in the ABRdel group were considerably less variable than those observed following either standard needle-based injection route. Mean titers after three immunizations were 12,160 for the ABRdel group, compared to 820 following IM injection and 4800 via ID injection. Notably, the ABRdel approach was the most effective delivery route following two immunizations; 100% (10/10) of animals treated via ABRdel seroconverted after two immunizations, compared to 40% (4/10) via the IM route and 50% (5/10) via ID injection. None of the animals administered plasmid DNA topically in the absence of abrasion mounted a detectable antibody response. Further characterization of the antibody isotypes revealed that administration of DNA vaccines using the microabrader device according to the ABRdel protocol induces a similar mixed response as standard needle-based IM and ID injections, consisting of both IgG1 and IgG2a. These results are in contrast to previously described epidermal vaccinations using the gene gun, in which antibody responses consisted exclusively of IgG 1 in the absence of IgG2a (e.g., see McCluskie, MJ et al., Molecular Medicine 5:287, 1999). In addition, delivery of plasmid DNA via microabraders induced potent cellular immune response, as measured by antigen-specific cytotoxic T cell stimulation.

### EXAMPLE 5

### DELIVERY OF ALLERGENS VIA MICROABRADER DEVICE

Histamine dihydrochloride (2.5 mg) was administered to the skin of anaesthetized swine by simultaneous abrasion and delivery using a microabrader device, as described in Example 1 (ABRdel; 4 passes of the device across the skin surface). The histamine was formulated either as a liquid or as a lyophilized powder, which was coated onto the surface of the abrading surface and reconstituted in water directly on the skin at the time of application. For comparison, histamine solution was placed as a droplet onto the surface of the skin, immediately after which a tine-like device was placed in contact with this solution and used to puncture the skin. This tine-like device consisted of seven metal 34 g needles of 1 mm length, similar to commercially available devices used in allergen testing. Adjacent skin sites were treated with the microabrader device or the tine-like puncturing device in the absence of histamine in order to monitor skin reactions due to the devices rather than the effects of histamine. Additional controls included skin sites treated with histamine topically in the absence of abrasion or puncture. Skin sites were monitored for immediate inflammatory reactions including redness, swelling and the appearance of a wheal-and-flare.

Vigorous inflammatory reactions were observed at skin sites treated with histamine via the microabrader device. Severe erythema and swelling (up to 2 mm of raised tissue) were observed across the entire area of histamine treated skin, whereas sites treated with the device in the absence of histamine displayed only mild redness along the path of abrasion in the complete absence of swelling. Similarly intense reactions were observed with both liquid and reconstituted powder histamine formulations. Skin sites treated with the histamine solution using the tine-like puncturing device also displayed severe erythema and swelling, although the response was localized to the points of contact of the tines and the immediate surrounding area. Skin sites treated topically with histamine solution in the absence of abrasion or puncture were not inflamed and appeared indistinguishable from normal, untreated skin.

Histamine dihydrochloride is used in the art as a model system for evaluation of peptide and polypeptide allergens. These results indicate that the described protocol of simultaneous abrasion and delivery can be effectively used for the topical administration of allergens which are amino acids or amino acid derivatives, and predict similar results for delivery of peptide or polypeptide allergens. Benefits of allergen delivery by microabrasion compared to skin puncture include distribution of the substance to a wider surface area of the skin, thus increasing the reactogenic site compared to the localized distribution accomplished using puncture with tine-like devices. The increased area of distribution, combined with better targeting of the highly immune-stimulatory epidermal tissue may increase the sensitivity of allergen testing compared to current tine-based skin puncture testing methods. In addition, by targeting the shallow epidermal tissue above the capillary beds and peripheral nerve net, delivery according to the current invention is likely to be less invasive and safer than current testing methods.

### Example 5B

In order to illustrate the differences between the present invention and what was previously known in the art (U.S. Patent No. 3,289,670) a number of experiments were performed. In a first set of experiments (Group A), plasmid DNA encoding the firefly luciferase reporter gene was delivered to a number of Brown-Norway rats by various methods and the results noted. The results of the Group A experiments show that the use of the present invention provides unexpected improvement in genetic expression over other methods. In a second set of experiments (Group B), histamine diphosphate was delivered to a number of weanling female Yorkshire pigs and the results noted. The results of the Group B experiments show that the use of the present invention provides an unexpected improvement in allergenic response over other methods.

### Group A Experiments

In each of the experiments in this group, 20 µg of plasmid DNA encoding the reporter gene, firefly luciferase, was administered in a total volume of 10 µl to Brown-Norway rats. A 30 gauge needle with a 1 cc syringe was used according to the Mantoux-style injection technique whereby the needle is inserted parallel to the skin surface. to deliver the injection intradermally.

In Group 2A, a microabrader device, as described in Example 1, except substituting a plastic abrading surface for the silicon abrading surface, was used according to the ABRdel protocol. The DNA solution was first applied in droplet form to the shaved skin of the rats. The microabrader device was then positioned onto the DNA solution and the skin. Thereafter, the microabrader device was used to simultaneously abrade the skin and deliver the DNA into the skin. To abrade the skin, the microabrader device was laterally moved over the skin 4 times (2 times each in alternating directions) across an area of approximately 1-1.5 cm². The center 1 cm² of the treatment site was collected for analysis.

In Groups 3A, 4A and 5A, a tine device (Greer Laboratories, Lenoir, NC, catalog number GP-1) consisting of a cluster of 6 substantially identical pointed elements arranged in a circle with a diameter of approximately 0.19 cm was used in accordance with the teachings of U.S. Patent No. 3,289,670. The device was loaded with the plasmid DNA solution by dipping the tines into a 10 µl droplet of the solution that essentially suspended all of the solution between the cluster of pointed elements (tines) by capillary action.

In Group 3A, the tine device was pressed against skin and scratched across an area of approximately 0.06 cm² being careful not to insert the tines so deep as to draw blood. The device was moved along a length of 1/8 inch (0.3175 cm) to provide a scratch with an area of approximately 0.06 cm².

In Group 4A, the tine device was pressed against skin and moved across an area of approximately 1cm² being careful not to insert the tines so as to draw blood. The device was moved along a length of 1 cm. Then, the device was removed from the skin and pressed against the skin adjacent to the original treatment site. The device was again moved along a length of 1 cm. This process was repeated until a full 1 cm² area of skin was treated.

In Group 5A, the tine device was pressed against skin being careful not to insert the tines so deep as to draw blood. The device was not used to scratch the skin; rather, the device was removed from the skin immediately after pressing against skin once.

In Group 6A, plasmid DNA was directly applied in droplet form to the shaved skin of the rats. Using a pipette tip, the droplet was then spread evenly across a 1 cm² area taking care not to scratch or abrade the skin.

The area of skin comprising each of the delivery sites was excised 24 hours post delivery, homogenized, and processed for luciferase activity using the Luciferase Assay System (Promega, Madison, WI). To account for differences in the total amount of tissue collected between groups, luciferase activity was normalized for total protein content in tissue specimens as determined by BCA assay (Pierce, Rockford, IL) and is expressed as Relative Light Units (RLU) per mg of total protein.

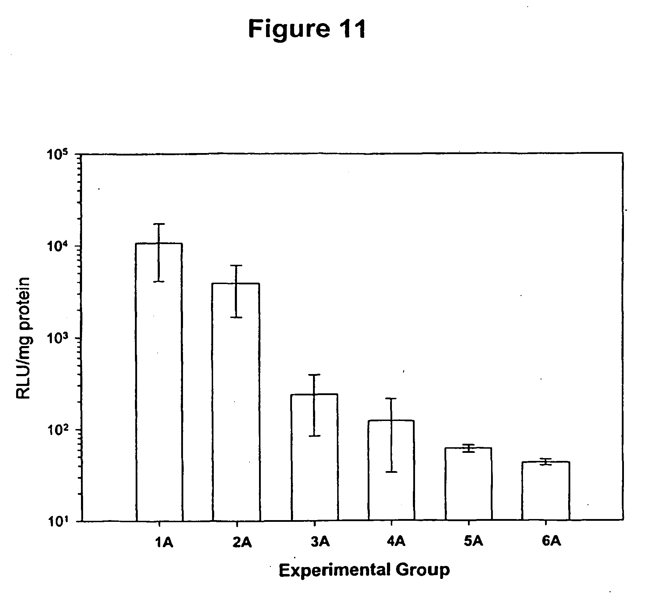

Figure 11 displays mean luciferase activity in skin samples obtained from the rats treated using the devices and methods as described above (n=4 per group) ± Standard Error of the Mean. Luciferase activity was strongest in the Group 1A at 10,720 RLU/mg. Delivery using the microabrader device (Group 2A) also resulted in strong luciferase activity in excised skin samples (3,880 RLU/mg). In contrast, delivery using the tine devices resulted in little to no increase in luciferase activity as compared to the topical control group. Luciferase activity was 237 RLU/mg when the tine device was used to scratch an area of approximately 0.06cm² (Group 2A) compared to 122 RLU/mg when used to scratch an area of approximately 1cm² (Group 3A), and 61 RLU/mg when pressed against skin without lateral movement (Group 5A). Topical application of the DNA plasmid in the absence of a delivery device (Group 6A) also failed to induce significant luciferase activity in skin (43 RLU/mg).

Thus, administration of plasmid DNA using a microabrader device and method of delivery as described in the Application results in reporter gene activity at levels up to 32 times greater than those observed following delivery using a tine device and method of delivery as described in U.S. Patent No. 3,289,670. Furthermore, delivery using the microabrader device of the present invention results in reporter gene activity at levels up to 90 times greater than those observed following delivery using a tine device as described in U.S. Patent No. 3,289,670 and pressed against the skin or following unassisted topical application. In addition to the above, visual inspection after administration of the substance by way of the method and device described in Group 3A-5A (tine device), revealed a substantial amount of substance remained suspended between the cluster of tines, whereas by contrast, the method and device of (Group 2A) appeared to retain substantially less of the substance.

### Group B Experiments

In each of the experiments in this group, 10 µl of a 276 mg/ml histamine diphosphate (Sigma, St. Louis, MO) solution (100 mg/ml histamine) was administered to weanling female Yorkshire pigs.

In Group 1B, a microabrader device as described in connection with the Group A experiment was used. The histamine solution was first applied in droplet form to the shaved skin of the pigs. The microabrader device was then positioned onto the histamine solution and the skin and used to simultaneously abrade the skin and deliver the histamine into the skin. To abrade the skin, the microabrader device was laterally moved over the skin 6 times (3 times each in alternating directions) across an area of approximately 1-1.5 cm².

In Group 2B, a tine device as described in connection with the Group 3A-5A experiments was used. The device was loaded with the histamine solution by dipping the tines into a 10 µL droplet of the solution that essentially suspended all of the solution between the cluster of tines by capillary action. Using slight pressure, the loaded device was then pressed against the shaved skin, being careful not to insert the tines so deep as to draw blood. The device was then moved along a length of 1/8 inch (0.3175 cm) to provide a scratch with an area of approximately 0.06cm².

In Group 3B, a tine device as described in connection with the Group 3A - 5A experiments was used. The tine device was pressed against the shaved skin and moved across an area of approximately 1 cm² being careful not to insert the tines so deep as to draw blood. The device was moved along a length of 1 cm. Then, the device was removed from the skin and pressed against the skin adjacent to the original treatment site. The device was again moved along a length of 1 cm. This process was repeated until a full 1 cm² area of skin was treated.

In Group 4B, a tine device as described in connection with the Group 3A - 5A experiments was used. The tine device was pressed against skin being careful not to insert the tines so deep as to draw blood. The device was not used to scratch the skin; rather, the device was removed from the skin immediately after pressing against skin once.

Control experiments were conducted using the methods described above, except without the application of the histamine solution.

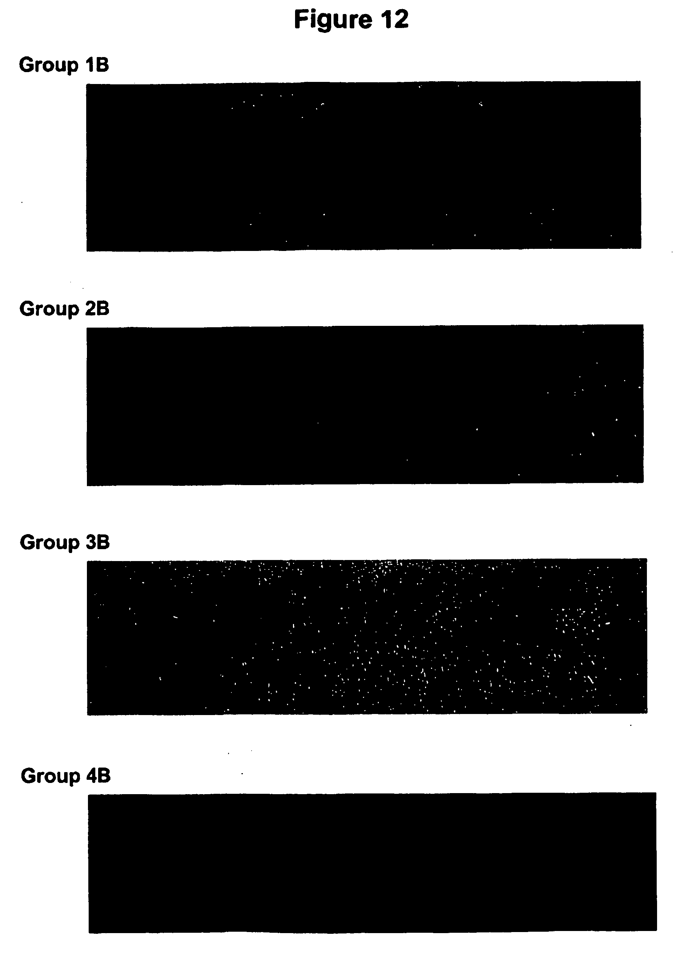

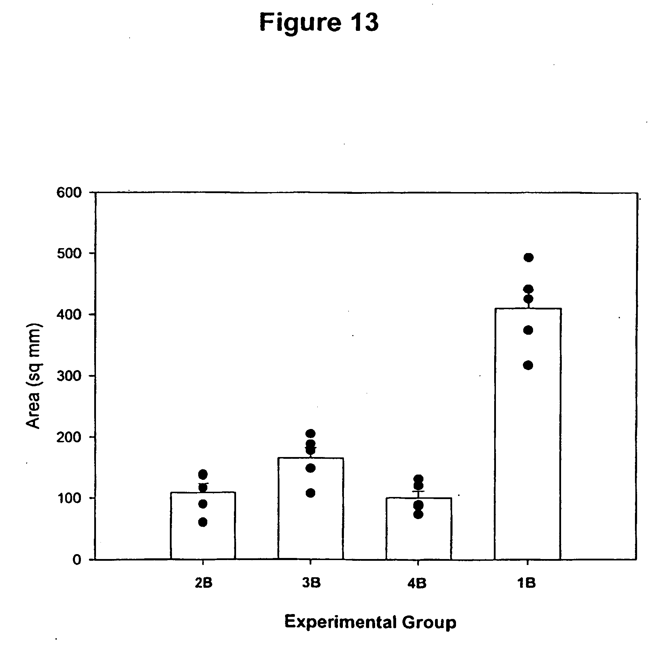

Skin sites were observed for redness and swelling at 20 minutes post treatment. The swollen skin sites were measured vertically and horizontally at the longest and widest points of the reaction using digital calipers. Although the reaction sites were not of uniform geometry, an estimate of the area was made by multiplying the vertical and horizontal measurements. Figure 12 displays photos of skin reactions. The results indicate that application of the histamine solution with the microabrader device of the instant invention (Group 1B) results in a greater area of histamine-induced swelling than the corresponding reactions induced by the tine device disclosed in U.S. Pat. No. 3,289,670 (Groups 2B-4B). Notably, while delivery of histamine with the microabrader device resulted in significant skin reactions, control sites treated with the device alone were completely clear at 20 min and showed no evidence of swelling or redness. In contrast, the tine devices applied to the skin without histamine resulted in considerable swelling and redness, making it difficult to distinguish effects of the device alone from the effects of the histamine. Figure 13 displays the relative area of tissue swelling for each group obtained after subtracting the swelling measurements observed from use of the device only without histamine. The results indicate that the mean area of histamine-induced swelling is up to 4 times greater when administered using the microabrader device of the present invention (Group 1 B) as compared to the tine device of U.S. Patent No. 3,289,670 (Groups 2B-4B). In addition to the above, visual inspection after administration of the substance by way of the method and device described in (Group 2B-4B) (tine device), revealed a substantial amount of substance remained suspended between the cluster of tines. In contrast, the method and device of (Group 1B) appeared to retain substantially less of the substance.

### EXAMPLE 6

### MICROABRADER DEVICES COMPRISING PLASTIC ABRADING SURFACES

The prior art describes Micro-Electro Mechanical Systems (MEMS)-based methods to fabricate structurally precise abrading surfaces from silicon. Microabrader devices comprising plastic abrading surfaces have several advantages over microabrader devices comprising silicon abrading surfaces including ease of manufacture, low cost and high reproducibility. Although such plastic abrading surfaces appear to have similar features as the silicon originals it was not known whether they would perform to the same capacity in vivo.

The following example shows the utility of microabrader devices comprising plastic abrading surface.

### EXAMPLE 6a

### DISRUPTION OF SKIN BARRIER FUNCTION

The outer 10-20 µm of skin, the stratum corneum layer, represents an effective physical and chemical barrier. An intact stratum corneum prevents passive topical absorption of vaccines and other drug substances into and across the skin. To compare the efficacy of microabrader devices comprising plastic and silicon abrading surfaces in disrupting this skin barrier, trans-epidermal water loss (TEWL) was measured on rat skin following treatment with the microabrader devices, as described in Example 1.

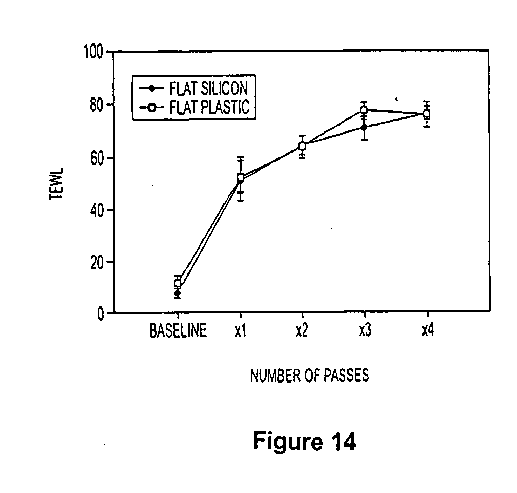

The treatment process consisted of laterally passing the microabrader device a variable number of times across a shaved section of the caudal dorsum of anaesthetized animals. TEWL readings were made before treatment and after each passage of the microabrader device using standard instrumentation (cyberDERM, Media, Pennsylvania). A total of n=4 per group were evaluated. Figure 14 presents mean TEWL measurements and standard errors.

The results demonstrate that the skin barrier function is disrupted to a similar extent using microabraders comprising the plastic and silicon abrading surfaces. Significant increases in TEWL were observed following a single pass of each device across the skin and continued to increase with additional passes. Both devices performed identically in disrupting this barrier regardless of the number of passes. In contrast, other types of devices lacking the micro-architecture as found on the microabraders (e.g., toothbrush) did not cause an increase in TEWL with this number of passes (data not shown).

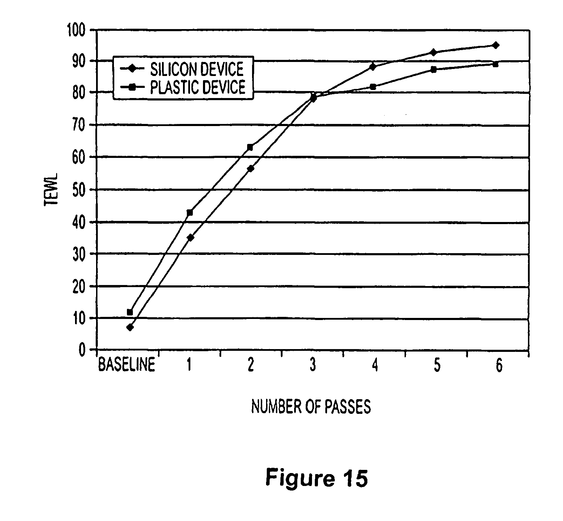

The microabrader devices were also tested in a swine model. The outer stratum corneum layer of pig skin is approximately 5-10 µm thicker than the corresponding layer in rat skin. Nonetheless, both silicon and plastic abrading surfaces were effective in disrupting this barrier, resulting in significant TEWL after as little as one pass of the device across the skin and continued to increase with additional passes (Figure 15; n=3 sites per condition on a single pig). As above, identical results were obtained with the 2 device types.

Histological analyses of stratum corneum disruption and penetration of fluorescent beads in pigs revealed similar results when comparing silicon and plastic abrading surfaces. In this example, a solution of fluorescent beads was applied to a skin site that was pre-treated by 2 lateral passes of the microabrader device. After topical application of the bead solution, the device was cleaned in alcohol, dried then placed in contact with the bead solution on the skin surface and rubbed across the skin an additional 2 times. Histologic analysis of recovered application sites revealed a similar pattern and extent of stratum corneum disruption and bead distribution following delivery via the silicon and plastic abrading surfaces. Beads were present across the surface of the treated skin sites and showed evidence of epidermal penetration.

### EXAMPLE 6b

### DELIVERY AND EXPRESSION OF PLASMID DNA ENCODING A REPORTER GENE

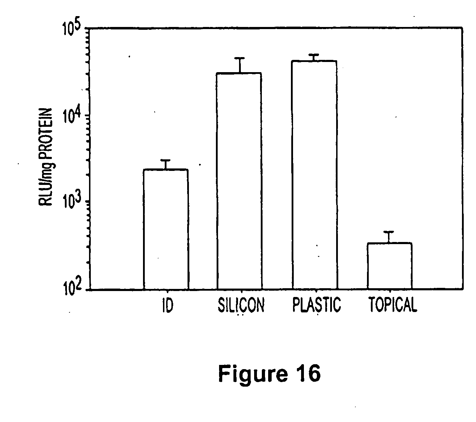

Plasmid DNA encoding the reporter gene, firefly luciferase, was administered to mice using microabrader devices comprising plastic or silicon abrading surfaces (Figure 16). The administration protocol was according to the ABRdel protocol as described in Example 1. A total of 37.5 µg of naked plasmid DNA was administered in 25 µl volume. Controls included ID injection with standard needle and syringe and topical application to shaved skin without use of a microabrader device (n=3 mice per group).

The results demonstrate that microabraders comprising the plastic abrading surfaces are very effective in the delivery of plasmid DNA resulting in significant levels of localized gene expression in skin (Figure 16). Mean luciferase activity in the group receiving plasmid DNA via the microabrader comprising a plastic abrading surface was 140-times greater than controls administered DNA topically without aid of a microabrader device. Administration via the microabraders comprising a silicon abrading surface resulted in similar high expression with mean activity approximately 100-times that of controls. Higher levels of luciferase activity were observed in both microabrader groups compared to standard needle-based ID injection (mean RLU/mg: plastic abrading surface, 43,688; silicon abrading surface, 31,034; ID injection, 2,214; Topical, 313).

Overall, the results demonstrate that microabraders comprising plastic abrading surfaces are at least as effective as microabraders comprising silicon abrading surfaces in the delivery and expression of plasmid DNA. In addition, microabrader devices are more effective than the standard needle in delivering plasmid DNA to skin, resulting in greater levels of gene expression.

### EXAMPLE 6c

### DELIVERY OF DNA VACCINE

The data presented in Figure 10 was re-plotted as a line graph and presented along with the results obtained from a separate set of mice (n=3 per group) immunized according to the same methods as described in Example 1, except using microabrader devices comprising a plastic abrading surface.

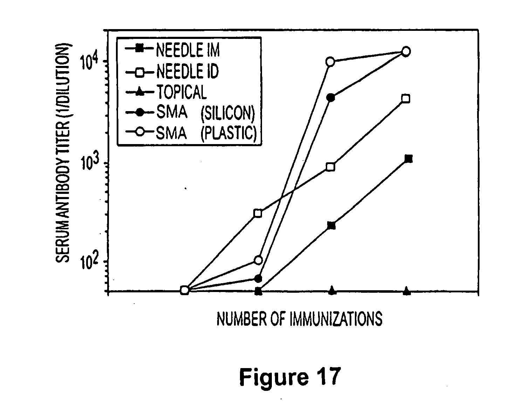

The results demonstrate that microabraders comprising plastic abrading surfaces are as effective as those comprising silicon abrading surfaces in inducing antigen-specific immune responses (Figure 17). Serum antibody titers induced via both microabrader devices were stronger than those induced by standard needle-based ID and IM injections. No significant responses were observed following topical application in the absence of an microabrader device, demonstrating that the device and method of the present invention enables topical immunization.

### EXAMPLE 7:

### ANTIBODY RESPONSE FOLLOWING DELIVERY OF DNA VACCINE FOR INFLUENZA WITHOUT ADDED ADJUVANT VIA MICROABRADER DEVICE

To further investigate the utility of delivering DNA vaccines by the device and method of the present invention, Brown-Norway rats were immunized with plasmid DNA encoding influenza virus hemagglutinin (HA) from strain A/PR/8/34 (plasmid provided by Dr. Harriet Robinson, Emory University School of Medicine, Atlanta, GA). Rats (n=3 per group) were immunized three times (days 0, 21 and 42) with plasmid DNA in PBS solution (50µg per rat in 50µl volume). Vaccine was administered using a microabrader device comprising a plastic abrading surface as described in Example 6, and according to the ABRdel protocol, as described in Example 1. Alternatively, the vaccine was injected ID or IM using needles. As a negative control, DNA was applied topically to shaved, but otherwise untreated skin. Sera were collected at weeks 3, 5, 8 and 11 and analyzed for the presence of influenza-specific antibodies by ELISA. Briefly, microtiter wells (Nalge Nunc, Rochester, NY) were coated with 0.1 µg of whole inactivated influenza virus (A/PR/8/34; Charles River SPAFAS, North Franklin, CT) overnight at 4°C. After blocking for 1hr at 37 °C in PBS plus 5% skim milk, plates were incubated with serial dilutions of test sera for 1 hr at 37 °C. Plates were then washed and further incubated with horse radish peroxidase conjugated anti-rat IgG, H+L chain (Southern Biotech, Birmingham, AL) for 30 min at 37°C and were then developed using TMB substrate (Sigma, St. Louis, MO). Absorbance measurements (A₄₅₀) were read on a Tecan Sunrise™ plate reader (Tecan, RTP, NC).

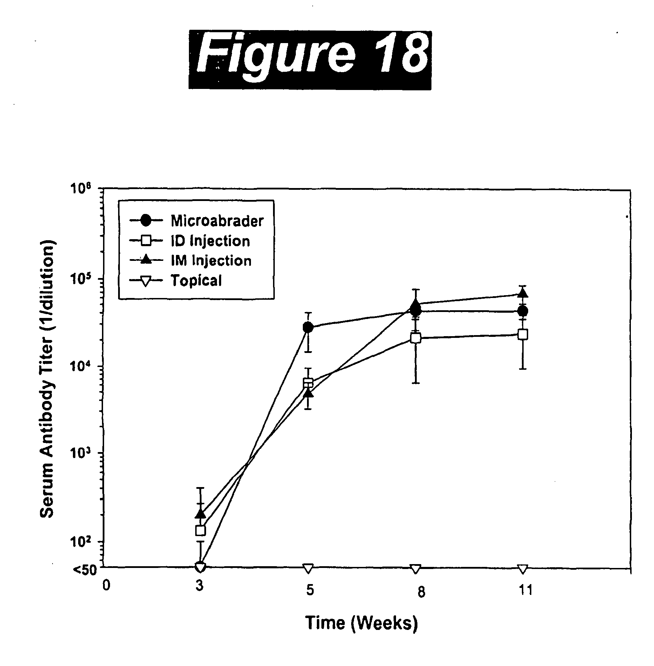

The results (Figure 18) demonstrate that serum antibody responses induced following delivery of naked plasmid DNA vaccine via the microabrader devices are as strong or stronger than those induced by ID or IM injection.

### EXAMPLE 8

### ANTIBODY RESPONSE FOLLOWING DELIVERY OF DNA VACCINE FOR INFLUENZA WITH ADDED ADJUVANT VIA MICROABRADER DEVICE

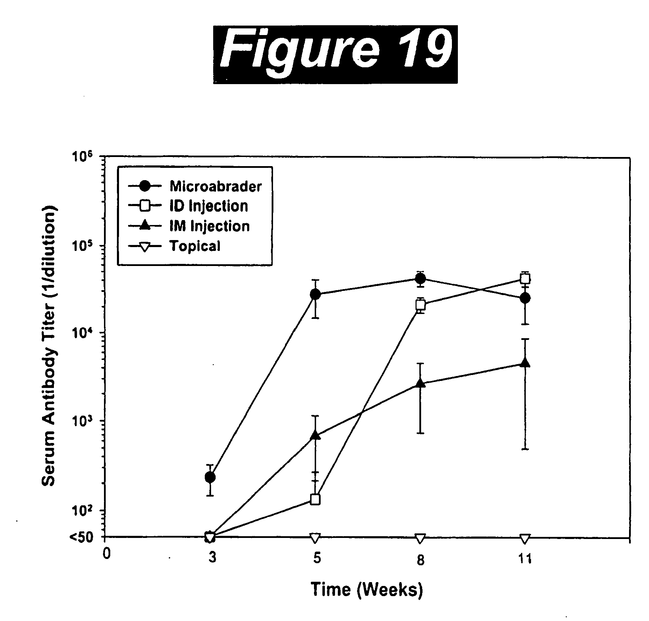

To further investigate delivery of adjuvanted genetic vaccines by the device and method of the present invention, the influenza HA-encoding plasmid DNA described in Example 7 was prepared using the MPL + TDM Ribi adjuvant system (RIBI Immunochemicals, Hamilton, MT) according to the manufacturer's instructions. Rats (n=3 per group) were immunized and analyzed for influenza-specific serum antibody as described in Example 7. The results (Figure 19) demonstrate that serum antibody responses induced following delivery of adjuvanted plasmid DNA vaccine via the microabrader devices are stronger and quicker to develop than those induced by ID or IM injection.

### EXAMPLE 9

### ANTIBODY RESPONSE FOLLOWING DELIVERY OF "PRIME-BOOST" INFLUENZA VACCINE WITHOUT ADDED ADJUVANT VIA MICROABRADER DEVICE

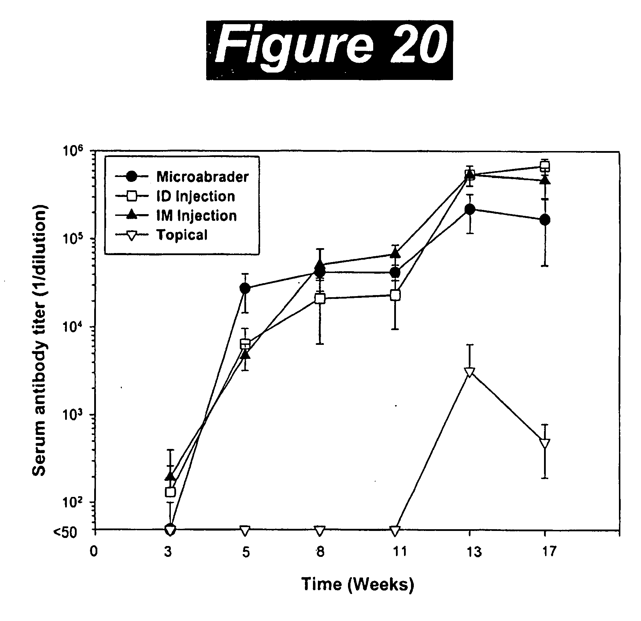

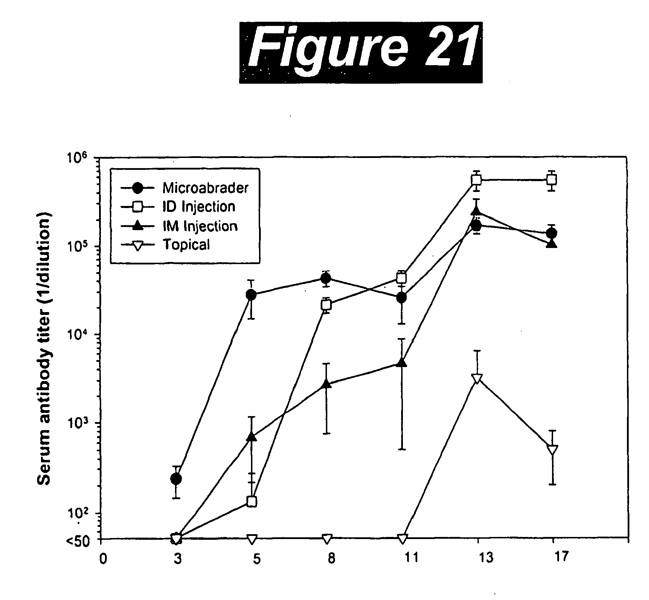

A recently developed vaccination approach for numerous diseases, including HIV, is the so-called "prime-boost" approach wherein the initial "priming" immunizations and secondary "boosters" employ different vaccine classes (Immunology Today, Apr 21(4): 163-165, 2000). For example, one may prime with a plasmid DNA version of the vaccine followed by a subsequent boost with a subunit protein, inactivated virus or vectored DNA preparation. To investigate delivery by the device and method of the present invention, rats from Example 7 were boosted at week 11 with whole inactivated influenza virus (Figure 20). (A/PR/8/34) 100µg in 50µl volume of PBS). (Virus obtained from Charles River SPAFAS, North Franklin, CT.) The results indicate a similar booster effect in all groups. Thus, administration of vaccines according to a "prime-boost" strategy using microabrader devices results in the stimulation of immune responses at levels that are at least as strong as those induced by ID or IM injection.
In a similar experiment, following the use of vaccine with adjuvant, rats from Example 8 were boosted at week 11 with whole inactivated influenza virus as described above. The results (Figure 21) indicate a similar booster effect in all groups. It was only after this boost that the immune response in the IM group was comparable to that induced by the microabrader. Thus, administration of vaccines according to a "prime-boost" strategy including adjuvants using microabrader devices results in the stimulation of immune responses at levels that are at least as strong as those induced by ID or IM injection.

### EXAMPLE 10

### ANTIBODY RESPONSE FOLLOWING DELIVERY OF RABIES VACCINE VIA MICROABRADER DEVICE

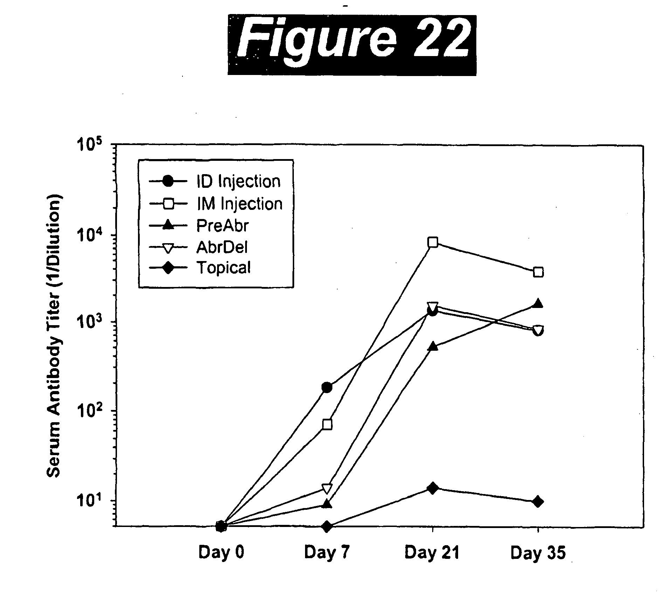

Rats were immunized at days 0, 7 and 21 with 25 µl rabies vaccine (Aventis Imovax). Vaccine was administered using a microabrader device comprising a plastic abrading surface as described in Example 6, and according to either the preABR protocol or the ABRdel protocol, as described in Example 1. Alternatively, the vaccine was injected ID or IM using needles. As a negative control, DNA was applied topically to shaved, but otherwise untreated skin. Rats (n=5/group)were immunized at d0 d7 and d21. Sera were collected at d0 d7, d21 and d35 and analyzed for the presence of rabies-specific antibodies using the Rapid Fluorescence Focus Inhibition Assay at the University of Kansas. The results (Figure 22) demonstrate that rabies neutralizing antibody titers induced following delivery using microabraders is delayed compared to that induced by injection. By d21 and persisting out to d35, however, titers became comparable to those achieved by injection. Furthermore, similar levels of response were observed for both methods of delivery; ABRdel and preABR. Topical application of the vaccine to shaved but otherwise untreated skin failed to induce a significant response. Thus, microabraders enable topical delivery of inactivated viral vaccines and standard injectable vaccine formulations. This demonstrates compatibility of microabrader devices with conventional vaccines formulated for injection. In addition, for a whole inactivated virus vaccine preparation, the method of delivery with a microabrader device does not appear to have an effect on the strength of the immune response.

### EXAMPLE 11a

### ANTIBODY AND T CELL RESPONSES FOLLOWING DELIVERY OF HEPATITIS B VACCINE VIA MICROABRADER DEVICE

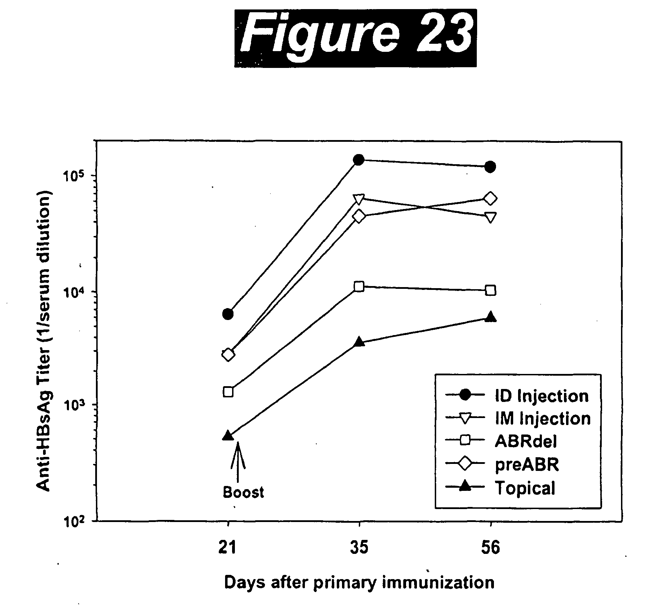

In another experiment, Hepatitis B surface antigen (HBsAg) protein subunit vaccine was administered to BALB/c mice by the following delivery routes:
1) Intramuscular (IM) injection using standard needle
2) Intradermal (ID) injection using standard needle
3) Microabrader as described in Example 1 used according to the "preABR" method as described in Example 1.
4) Microabrader as described in Example 1 used according to the "ABRdel" method as described in Example 1.
5) Topical application to shaved skin directly (no abrader was used; labeled "Topical" in Figure 23)

All animals (n=4 per group) received 2 immunizations, each consisting of 10µg HbsAg plus 10µg CpG-containing oligonucleotides as adjuvant. Immunizations were given at the onset of the experiment ("day 0", d0) and at d21. Mice were bled and analyzed for HbsAg-specific serum antibody titers by ELISA at d21, d35 and d56. The results (Figure 23) demonstrate that treatment with a microabrader device enables topical immunization Antibody responses in the preABR group were significantly greater than the corresponding very weak responses observed in the topical control group. The magnitude of response induced by pre-treatment with the microabrader device was comparable to that observed following direct IM or ID injection with a standard needle. Notably, in contrast to the results obtained for nucleic acids (Example 1), delivery of HBsAg by simultaneous abrasion and delivery (ABRdel) elicited a weaker response. Thus, the most appropriate method of delivery using microabrader devices depends, at least in part, on the type of substance to be delivered. HBsAg represents a subunit vaccine consisting of protein monomers that self-assemble into virus-like particles. The results depicted in Example 11 demonstrate that this class of vaccine is best administered by pre-treatment with the microabrader, although significant responses could also still be induced via the "simultaneous abrasion and delivery" method.

### EXAMPLE 11b

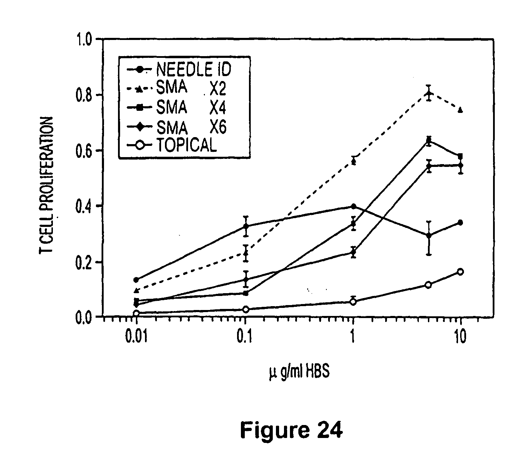

In another experiment, HBsAg protein subunit vaccine was administered to BALB/c mice by the following delivery routes:
1) Intradermal (ID) injection using standard needle
2) Microabrader as described in Example 1 used according to the "preABR" method as described in Example 1, except limiting the number of passes of the microabrader device across the skin surface to two passes.
3) Microabrader as described in Example 1 used according to the "preABR" method as described in Example 1, except limiting the number of passes of the microabrader device across the skin surface to four passes.
4) Microabrader as described in Example 1 used according to the "preABR" method as described in Example 1 (6 passes across skin). Topical application to shaved skin directly (no abrader was used; labeled "Topical" in Figure 24).

All animals (n=3 per group) received 1 immunization with 10µg HBsAg plus 10µg CpG-containing oligonucleotides. Ten days post-immunization, single cell suspensions were collected from draining lymph nodes (DLN) and re-stimulated in culture with the indicated doses of HBsAg. T cell proliferation was measured after 5 days of culture using a commercial MTS-based assay.

The results (Figure 24) demonstrate that pre-treatment with a microabrader device enables topical immunization. Strong T cell proliferative responses were observed in all groups treated with the microabrader, compared to very little to no response in the topical control group. The magnitude of response in the microabrader-treated groups were greater at most doses than the corresponding responses observed following ID injection with a standard needle. Strongest responses were observed following only 2 passes of the device across skin. There was a minor drop in proliferative activity following 4 or 6 passes. Additional experiments showed a further drop with >6 passes of the device with a complete loss of activity following 10 passes (data not shown). These results suggest that there is an optimal number of passes of the device that must be determined to enable topical immunization with a given vaccine. In addition, these results suggest that a mild treatment protocol (as few as 2 passes) can in some cases be sufficient to disrupt the outer skin barrier and enable topical immunization.

### EXAMPLE 12

### T CELL RESPONSE FOLLOWING DELIVERY OF ADENOVIRAL VECTORED VACCINE FOR MELANOMA VIA MICROABRADER DEVICE

An adenovirus delivering DNA encoding gp100 (a melanoma tumor antigen), was tested using microabraders, topical, and ID delivery, *inter alia,* in a mouse melanoma model. The following experimental groups were investigated (n=8/group):
1) Vehicle only administered via the "ABRdel" protocol as described in Example 1, using microabrader devices as described in Example 1.
2) Adenoviral (Ad2) vectored vaccine encoding melanoma gp100 antigen administered via the "ABRdel" protocol as described in Example 1, using microabrader devices as described in Example 1.
3) Vehicle only administered via the "preABR" protocol as described in Example 1, using microabrader devices as described in Example 1.
4) Adenoviral (Ad2) vectored vaccine encoding melanoma gp100 antigen administered via the "preABR" protocol as described in Example 1, using microabrader devices as described in Example 1.
5) Adenoviral (Ad2) vectored vaccine encoding melanoma gp100 antigen administered topically to shaved but otherwise untreated skin.
6) Adenoviral (Ad2) vectored vaccine encoding melanoma gp100 antigen administered via ID injection using conventional needles.

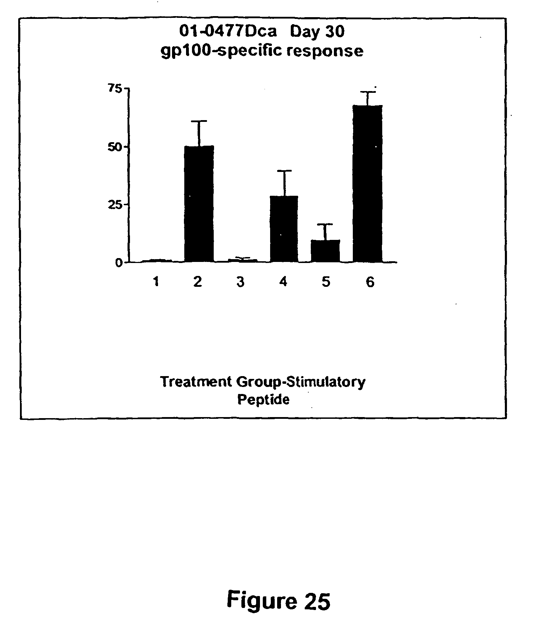

A total of 2x10⁹ adenovirus particles per mouse were administered. The gp100 specific cellular immune response was measured on day 30 following administration of the vaccine by ELISPOT assay of splenic interferon-gamma producing cells. The results are shown in Figure 25. Delivery using the microabraders according to the "ABRdel" protocol (Group 2) produced a significant response compared to topical delivery (Group 5), although it was somewhat weaker than that produced via ID injection (group 6). Notably, for this adenovirally-vectored vaccine, stronger cellular immune responses were observed in the "ABRdel" group (Group 2) as compared to the "preABR" group (Group 4). These results are similar to those observed for plasmid DNA (see Example 1). Thus, the most appropriate method of delivery using microabrader devices depends, at least in part, on the type of substance to be delivered. The Ad2 vector represents a live virus. The results depicted in Example 13 demonstrate that this class of vaccine is best administered by simultaneous abrasion and delivery, although detectable immune responses could also be induced by the "preABR" method.

Further description of suitable adenoviral vectors for use in vaccines can be found, *inter alia,* in U.S. Pat. No. 5,882,877.

### EXAMPLE 13

### ANTIBODY RESPONSE FOLLOWING DELIVERY OF RECOMBINANT PROTEIN SUBUNIT VACCINE FOR ANTHRAX VIA MICROABRADER DEVICE

In another experiment, mice were immunized with the recombinant protective antigen (rPA) of Bacillus anthracis. The rPA was provided by Dr. Robert Ulrich at the United States Army Medical Research in Infectious Diseases (USAMRIID). BALB/c mice (n=10/group) were immunized with 10µg of rPA in the presence of absence of additional adjuvants as detailed below:
Group 1: IM - rPA plus Alhydrogel (alum) adjuvant
Group 2: Microabrader, "preABR" - rPA (no adjuvant)
Group 3: Microabrader, "preABR" - rPA plus Alhydrogel (alum) adjuvant
Group 4: Microabader, "preABR" - rPA plus CpG-oligonucleotide adjuvant
Group 5: Microabrader, "ABRdel" - rPA (no adjuvant)
Group 6: Microabrader, "ABRdel" - rPA plus Alhydrogel (alum) adjuvant
Group 7: Microabader, "ABRdel" - rPA plus CpG-oligonucleotide adjuvant
Group 8: Topical - rPA (no adjuvant)
Group 9: Topical - rPA plus Alhydrogel (alum) adjuvant
Group 10: Topical - rPA plus CpG-oligonucleotide adjuvant

Mice were immunized on d0, d21 and d42. Sera were collected and analyzed for rPA-specific antibodies by ELISA at d21, d42 and d56. Results are summarized in Tables 1-3 below.

**Table 1: Anti-rPA serum antibody titers at d21 (1 dose of anthrax vaccine). Titers of individual animals (n=10) and mean for each group are indicated.**

| Group: | **1) IM** | **2) preABR** | **3) preABR** | **4) preABR** | **5) ABRdel** | **6) ABRdel** | **7) ABRdel** | **8) Topical** | **9) Topical** | **10) Topical** |
|---|---|---|---|---|---|---|---|---|---|---|
| Adjuvant: | **alum** | **none** | **alum** | **CpG** | **none** | **alum** | **CpG** | **none** | **alum** | **CpG** |
| | **50** | **50** | <50 | <50 | **50** | <50 | <50 | **50** | <50 | <50 |
| | **50** | <50 | <50 | **50** | <50 | <50 | <50 | <50 | <50 | <50 |
| | <50 | <50 | <50 | <50 | <50 | <50 | <50 | <50 | <50 | <50 |
| | <50 | <50 | <50 | <50 | <50 | <50 | <50 | <50 | <50 | <50 |
| | <50 | <50 | **50** | **50** | <50 | <50 | <50 | <50 | <50 | <50 |
| | <50 | <50 | <50 | <50 | <50 | <50 | <50 | <50 | <50 | <50 |
| | <50 | <50 | **200** | <50 | <50 | <50 | <50 | <50 | <50 | <50 |
| | <50 | <50 | <50 | **200** | <50 | <50 | <50 | <50 | <50 | <50 |
| | <50 | <50 | **100** | <50 | <50 | <50 | <50 | <50 | <50 | <50 |
| | <50 | <50 | <50 | <50 | <50 | <50 | <50 | <50 | <50 | <50 |
| Mean: | 10 | 5 | 35 | 30 | 5 | <50 | <50 | 5 | <50 | <50 |

**Table 2: Anti-rPa serum antibody titers at d42 (2 doses of anthrax vaccine). Titers of individual animals (n=10) and mean for each group are indicated.**

| Group: | **1) IM** | **2) preABR** | **3) preABR** | **4) preABR** | **5) ABRdel** | **6) ABRdel** | **7) ASRdel** | **8) Topical** | **9) Topical** | **10) Topical** |
|---|---|---|---|---|---|---|---|---|---|---|
| Adjuvant: | **alum** | **none** | **alum** | **CpG** | **none** | **alum** | **CpG** | **none** | **alum** | **CpG** |
| | **51200** | **12800** | **6400** | **6400** | **6400** | **1600** | **6400** | **400** | **50** | **50** |
| | **12800** | **12800** | **25600** | **25600** | **3200** | **6400** | **1600** | <50 | <50 | <50 |
| | **25600** | **12800** | **25600** | **12800** | **400** | **3200** | **3200** | <50 | <50 | <50 |
| | **3200** | **12800** | **6400** | **6400** | **6400** | **6400** | **3200** | <50 | <50 | <50 |
| | **6400** | **6400** | **25600** | **51200** | **6400** | **6400** | **12800** | <50 | **3200** | <50 |
| | **25600** | **25600** | **6400** | **12800** | **1600** | **6400** | **6400** | <50 | <50 | **800** |
| | **3200** | **12800** | **25600** | **25600** | **3200** | **12800** | **6400** | <50 | <50 | <50 |
| | **3200** | **1600** | **3200** | **25600** | **6400** | **3200** | <50 | <50 | <50 | **6400** |
| | **25600** | **6400** | **6400** | **102400** | **3200** | **6400** | **6400** | **12800** | <50 | <50 |
| | | **12800** | **25600** | **12800** | | | **12800** | <50 | | **3200** |
| Mean: | 17,422 | 11.680 | 15,680 | 28,160 | 4,133 | 5,867 | 6,578 | 1,320 | 361 | 1,045 |

**Table 3: Anti-rPa serum antibody titers at d42 (3 doses of anthrax vaccine). Titers of individual animals (n=10) and mean for each group are indicated.**

| Group: | **1) IM** | **2) preABR** | **3) preABR** | **4) preABR** | **5) ABRdel** | **6) ABRdel** | **7) ABRdel** | **8) Topical** | **9) Topical** | **10) Topical** |
|---|---|---|---|---|---|---|---|---|---|---|
| Adjuvant: | **alum** | **none** | **alum** | **CpG** | **none** | **alum** | **CpG** | **none** | **alum** | **CpG** |
| | **25600** | **51200** | **51200** | **51200** | **25600** | **25600** | **25600** | **12800** | **200** | **3200** |
| | **51200** | **25600** | **51200** | **51200** | **25600** | **25600** | **51200** | <50 | 50 | **<50** |
| | **25600** | **51200** | **25600** | **204800** | **25600** | **102400** | **25600** | <50 | **1600** | **6400** |
| | **25600** | **12800** | **51200** | **102400** | **51200** | **51200** | **51200** | <50 | **1600** | <50 |
| | **102400** | **51200** | **51200** | **51200** | **25600** | **25600** | **102400** | <50 | **3200** | <50 |
| | **51200** | **25600** | **25600** | **51200** | **25600** | **25600** | **51200** | <50 | <50 | **6400** |
| | **51200** | **25600** | **51200** | **51200** | **12800** | **51200** | **51200** | **3200** | **100** | **100** |
| | **102400** | **12800** | **12800** | **51200** | **51200** | **51200** | **25600** | **50** | **400** | **12800** |
| | | **25600** | **25600** | **51200** | **51200** | | **102400** | **12800** | **100** | **800** |
| | | **25600** | **25600** | **51200** | | | **204800** | <50 | | **6400** |
| Mean: | 54,400 | 30,720 | 37,120 | 71,680 | 32,711 | 44,800 | 69,120 | 2,885 | 806 | 3,610 |

(In cases where fewer than 10 values are given, animals died.)

The results demonstrate that significant serum antibody titers are achieved using microabraders to deliver a recombinant subunit vaccine for anthrax. Antibody titers generated via delivery using microabraders are at least as strong as those generated via the conventional route of IM injection. Topical administration without microabraders induces a comparatively weak response in some animals. Similar to the results presented in Example 11 a, the "preABR" protocol induced greater immune responses than the "ABRdel" protocol, after just 1 or 2 doses of vaccine (Tables 1 and 2). By the third dose, however, titers were comparable among these groups (Table 3). Thus, the most appropriate method of delivery using microabrader devices depends, at least in part, on the type of substance to be delivered. rPA represents a subunit vaccine consisting of recombinant protein. The results depicted in Tables 1-3 demonstrate that this class off vaccine is best administered by pre-treatment with the microabrader, although significant responses could also ultimately be induced via the "simultaneous abrasion and delivery" method.

Furthermore, the results demonstrate that the device and methods of the invention are compatible with multiple types of vaccine adjuvants including, for example, alum and CpG-containing oligonucleotides.

These results demonstrate that microabraders and techniques of the present invention enable topical delivery of a wide variety of classes of vaccines and improve delivery in many cases as compared to conventional delivery methods using a standard needle and syringe.

## Claims

1. A device (2) for delivering a substance into skin comprising an abrading surface (5) coated with the substance and a reservoir containing a reconstituting liquid in fluid communication with the abrading surface (5).

2. The device (2) of claim 1, wherein the reservoir communicates with the abrading surface (5) via channels through microprotrusions (14) on the abrading surface (5).

3. The device (2) of claim 1, wherein the reservoir communicates with the abrading surface (5) via channels between microprotrusions (14) on the abrading surface (5).

4. The device (2) of claim 1, wherein the reservoir communicates with the abrading surface (5) by means of a porous material between the reservoir and the abrading surface (5).

5. The device (2) of claim 1, further comprising a base (4) comprising an abrading facet adapted to receive or integral with said abrading surface (5).

6. The device (2) of claim 5, wherein said abrading surface (5) projects from said abrading facet.

7. The device (2) of claim 5, further comprising a handle attachment facet (4c) suitable for attaching a handle (6) to said base (4).

8. The device (2) of claim 5, further comprising a handle (6) that is integral with or detachable from said base (4).

9. The device (2) of claim 8, wherein said handle (6) is removable.

10. The device (2) of claim 7, wherein said handle attachment facet (4c) and said abrading facet are disposed substantially parallel to one another on opposite sides of said base (4).

11. The device (2) of claim 7, wherein said base (4) comprises a smooth edge connecting said abrading facet to said handle attachment facet (4c).

12. The device (2) of claim 11, wherein said smooth edge forms an arc connecting said abrading facet to said handle attachment facet (4c).

13. The device (2) of claim 11, wherein said abrading facet is substantially circular of a first diameter, and said handle attachment facet (4c) is substantially circular of a second diameter, and wherein said second diameter is greater than said first diameter.

14. The device (2) of claim 1, wherein the abrading surface (5) is an array comprising a plurality of microprotrusions (14).

15. The device (2) of claim 14, wherein the substance is coated on the microprotrusions (14).

16. The device (2) of claim 14, wherein said microprotrusions (14) are frustoconical or frustopyramidal microprotrusions (14) comprising at least one scraping edge (20,22) and projecting from said abrading surface (5).

17. The device (2) of claim 14, wherein said microprotrusions (14) are at least partially coated with said substance to be delivered.

18. The device (2) of claim 14, wherein said microprotrusions (14) are of a length sufficient to penetrate into or through the stratum corneum layer of said skin.

19. The device (2) of claim 14, wherein said microprotrusions (14) comprise at least two scraping edges (20,22).

20. The device (2) of claim 14, wherein said microprotrusions (14) comprise at least three facets (16) and wherein the intersection of any two of said facets forms a scraping edge (20,22).

21. The device (2) of claim 14, wherein said microprotrusions (14) comprise a flat tip (18).

22. The device (2) of claim 14, wherein said microprotrusions (14) have microprotrusion bases and are constructed and arranged in a pattern such that the distance between the centers of said microprotrusion bases is at least two times the length of said microprotrusion.

23. The device (2) of claim 14, wherein said microprotrusions (14) have microprotrusion bases and are constructed and arranged in a pattern such that the distance between the centers of said microprotrusion bases is at least five times the length of said microprotrusion.

24. The device (2) of claim 22 or 23, wherein said bases of said microprotrusions (14) are spaced apart to form valleys between said microprotrusions (14).

25. The device (2) of claim 14, wherein said microprotrusions (14) are arranged in a pattern.

26. The device (2) of claim 25, wherein said pattern consists of rows and columns, or wherein said pattern is a uniform, circular or random pattern.

27. The device (2) of claim 14, wherein the length of said microprotrusions (14) is greater than the depth to which they penetrate into said skin.

28. The device (2) of claim 14, wherein the length of said microprotrusions (14) is
(i) from 5 to 500 microns,
(ii) from 30 to 300 microns,
(iii) from 75 to 250 microns, or
(iv) from 180 to 220 microns.

29. The device (2) of claim 1, wherein said abrading surface (5) is plastic or silicon.

30. The device (2) of claim 1, wherein said substance is a bioactive substance.

31. The device (2) of claim 30, wherein said bioactive substance is
(i) a medicament,
(ii) a vaccine,
(iii) an allergen, or
(iv) a gene therapeutic agent.

32. The device (2) of claim 31 (ii), wherein said vaccine comprises
(i) a live, attenuated virus or viral vector,
(ii) an inactivated or killed virus,
(iii) an inactivated or killed bacterium,
(iv) a nucleic acid,
(v) a nucleic acid, and a protein or peptide encoded by said nucleic acid,
(vi) a live, non-attenuated virus or bacteria,
(vii) a polysaccharide or polysaccharide-conjugate, or
(viii) a protein or peptide.

33. The device (2) of claim 31 (ii) or 32 (viii), wherein said vaccine further comprises an adjuvant.

34. The device (2) of any one of the preceding claims, wherein, in use, the reconstituting liquid is releasable to dissolve the substance as the device is applied to the skin for abrasion.

35. The device (2) of claim 5 or 6, wherein the base (4) is configured with a mushroom like-crown (4b) that curves upward and is truncated of the top.

36. The device (2) of any one of the preceding claims, wherein the device further comprises means for controlling the rate of delivery of the substance or reconstituting liquid.

37. A kit comprising at least one device (2) of claim 1.

## Patentansprüche

1. Vorrichtung (2) zur Zuführung einer Substanz in Haut, umfassend eine mit der Substanz beschichtete abradierende Fläche (5) und ein Reservoir, das eine rekonstituierende Flüssigkeit in Fluidverbindung mit der abradierenden Fläche (5) enthält.

2. Vorrichtung (2) nach Anspruch 1, wobei das Reservoir über Kanäle durch Mikrovorsprünge (14) auf der abradierenden Fläche (5) mit der abradierenden Fläche (5) in Verbindung steht.

3. Vorrichtung (2) nach Anspruch 1, wobei das Reservoir über Kanäle zwischen Mikrovorsprüngen (14) auf der abradierenden Fläche (5) mit der abradierenden Fläche (5) in Verbindung steht.

4. Vorrichtung (2) nach Anspruch 1, wobei das Reservoir mittels eines porösen Materials zwischen dem Reservoir und der abradierenden Fläche (5) mit der abradierenden Fläche in Verbindung steht.

5. Vorrichtung (2) nach Anspruch 1, ferner umfassend eine Basis (4), umfassend eine abradierende Facette, die dahingehend angepasst ist, die abradierende Fläche (5) aufzunehmen, oder die diese integriert enthält.

6. Vorrichtung (2) nach Anspruch 5, wobei die abradierende Fläche (5) von der abradierenden Facette vorspringt.

7. Vorrichtung (2) nach Anspruch 5, ferner umfassend eine Halterungsbefestigungsfacette (4c), die zur Anbringung einer Halterung (6) an die Basis (4) geeignet ist.

8. Vorrichtung (2) nach Anspruch 5, ferner umfassend eine Halterung (6), die in die Basis (4) integriert oder davon lösbar ist.

9. Vorrichtung (2) nach Anspruch 8, wobei die Halterung (6) lösbar ist.

10. Vorrichtung (2) nach Anspruch 7, wobei die Halterungsbefestigungsfacette (4c) und die abradierende Facette im wesentlichen parallel zueinander auf gegenüberliegenden Seiten der Basis (4) angeordnet sind.

11. Vorrichtung (2) nach Anspruch 7, wobei die Basis (4) einen glatten Rand umfasst, der die abradierende Facette mit der Halterungsbefestigungsfacette (4c) verbindet.

12. Vorrichtung (2) nach Anspruch 11, wobei der glatte Rand einen Bogen bildet, der die abradierende Facette mit der Halterungsbefestigungsfacette (4c) verbindet.

13. Vorrichtung (2) nach Anspruch 11, wobei die abradierende Facette im wesentlichen kreisförmig mit einem ersten Durchmesser ist und die Halterungsbefestigungsfacette (4c) im wesentlichen kreisförmig mit einem zweiten Durchmesser ist, und wobei der zweite Durchmesser größer ist als der erste Durchmesser.

14. Vorrichtung (2) nach Anspruch 1, wobei die abradierende Fläche (5) eine Anordnung ist, die mehrere Mikrovorsprünge (14) umfasst.

15. Vorrichtung (2) nach Anspruch 14, wobei die Substanz auf die Mikrovorsprünge (14) geschichtet ist.

16. Vorrichtung (2) nach Anspruch 14, wobei die Mikrovorsprünge (14) kegelstumpfförmige oder pyramidenstumpfförmige Mikrovorsprünge (14) sind, die mindestens eine schabende Kante (20, 22) umfassen und von der abradierenden Fläche (5) vorspringen.

17. Vorrichtung (2) nach Anspruch 14, wobei die Mikrovorsprünge (14) zumindest teilweise mit der zuzuführenden Substanz beschichtet sind.

18. Vorrichtung (2) nach Anspruch 14, wobei die Mikrovorsprünge (14) eine Länge aufweisen, die ausreicht, um in die Stratum-corneum-Schicht der Haut ein- oder hindurchzudringen.

19. Vorrichtung (2) nach Anspruch 14, wobei die Mikrovorsprünge (14) mindestens zwei schabende Kanten (20, 22) umfassen.

20. Vorrichtung (2) nach Anspruch 14, wobei die Mikrovorsprünge (14) mindestens drei Facetten (16) umfassen, und wobei der Überschneidungsbereich je zweier beliebiger Facetten eine schabende Kante (20, 22) bildet.

21. Vorrichtung (2) nach Anspruch 14, wobei die Mikrovorsprünge (14) eine flache Spitze (18) umfassen.

22. Vorrichtung (2) nach Anspruch 14, wobei die Mikrovorsprünge (14) Mikrovorsprungbasen aufweisen und in einem Muster ausgebildet und angeordnet sind, so dass der Abstand zwischen den Zentren der Mikrovorsprungbasen mindestens das Doppelte der Länge der Mikrovorsprünge beträgt.

23. Vorrichtung (2) nach Anspruch 14, wobei die Mikrovorsprünge (14) Mikrovorsprungbasen aufweisen und in einem Muster ausgebildet und angeordnet sind, so dass der Abstand zwischen den Zentren der Mikrovorsprungbasen mindestens das Fünffache der Länge der Mikrovorsprünge beträgt.

24. Vorrichtung (2) nach Anspruch 22 oder 23, wobei die Basen der Mikrovorsprünge (14) voneinander beabstandet sind, um Täler zwischen den Mikrovorsprüngen (14) zu bilden.

25. Vorrichtung (2) nach Anspruch 14, wobei die Mikrovorsprünge (14) in einem Muster angeordnet sind.

26. Vorrichtung (2) nach Anspruch von 25, wobei das Muster aus Reihen und Spalten besteht, oder wobei das Muster ein einheitliches, kreisförmiges oder zufälliges Muster ist.

27. Vorrichtung (2) nach Anspruch 14, wobei die Länge der Mikrovorsprünge (14) größer ist als die Tiefe, in die sie in die Haut eindringen.

28. Vorrichtung (2) nach Anspruch 14, wobei die Länge der Mikrovorsprünge (14)
(i) 5 bis 500 Mikrometer,
(ii) 30 bis 300 Mikrometer,
(iii) 75 bis 250 Mikrometer, oder
(iv) 180 bis 220 Mikrometer.
beträgt.

29. Vorrichtung (2) nach Anspruch 1, wobei die abradierende Fläche (5) Kunststoff oder Silizium ist.

30. Vorrichtung (2) nach Anspruch 1, wobei die Substanz eine bioaktive Substanz ist.

31. Vorrichtung (2) nach Anspruch 30, wobei die bioaktive Substanz
(i) ein Medikament,
(ii) ein Vakzin,
(iii) ein Allergen, oder
(iv) ein gentherapeutisches Mittel
ist.

32. Vorrichtung (2) nach Anspruch 31(ii), wobei das Vakzin
(i) einen lebenden, abgeschwächten Virus oder viralen Vektor,
(ii) einen inaktivierten oder abgetöteten Virus,
(iii) ein inaktiviertes oder abgetötetes Bakterium,
(iv) eine Nukleinsäure
(v) eine Nukleinsäure und ein von der Nukleinsäure kodiertes Protein oder Peptid,
(vi) ein lebendes, nicht abgeschwächtes Virus oder Bakteriien,
(vii) ein Polysaccharid oder Polysaccharid-Konjugat, oder
(viii) ein Protein oder Peptid
umfasst.

33. Vorrichtung (2) nach Anspruch 31(ii) oder 32(viii), wobei das Vakzin ferner ein Adjuvans umfasst.

34. Vorrichtung (2) nach einem der vorhergehenden Ansprüche, wobei die rekonstituierende Flüssigkeit bei Gebrauch freisetzbar ist, um die Substanz zu lösen, wenn die Vorrichtung zur Abrasion auf die Haut angewendet wird.

35. Vorrichtung (2) nach Anspruch 5 oder 6, wobei die Basis (4) mit einer pilzähnlichen Krone ausgebildet ist, die sich aufwärts wölbt und an der Spitze abgeschnitten ist.

36. Vorrichtung (2) nach einem der vorhergehenden Ansprüche, wobei die Vorrichtung ferner Mittel zur Kontrolle der Zuführungsgeschwindigkeit der Substanz oder der rekonstituierenden Flüssigkeit umfasst.

37. Kit, umfassend mindestens eine Vorrichtung (2) nach Anspruch 1.

## Revendications

1. Dispositif (2) pour administrer une substance dans la peau comprenant une surface abrasive (5) revêtue de la substance et un réservoir contenant un liquide reconstituant en communication fluidique avec la surface abrasive (5).

2. Dispositif (2) selon la revendication 1, dans lequel le réservoir communique avec la surface abrasive (5) par le biais de canaux à travers des microprotubérances (14) sur la surface abrasive (5).

3. Dispositif (2) selon la revendication 1, dans lequel le réservoir communique avec la surface abrasive (5) par le biais de canaux entre des microprotubérances (14) sur la surface abrasive (5).

4. Dispositif (2) selon la revendication 1, dans lequel le réservoir communique avec la surface abrasive (5) au moyen d'un matériau poreux entre le réservoir et la surface abrasive (5).

5. Dispositif (2) selon la revendication 1, comprenant en outre une base (4) comprenant une facette abrasive adaptée pour recevoir ou solidaire avec ladite surface abrasive (5).

6. Dispositif (2) selon la revendication 5, dans lequel ladite surface abrasive (5) fait saillie de ladite facette abrasive.

7. Dispositif (2) selon la revendication 5, comprenant en outre une facette de raccord de poignée (4c) appropriée pour raccorder une poignée (6) à ladite base (4).

8. Dispositif (2) selon la revendication 5, comprenant en outre une poignée (6) qui est solidaire de ou détachable de ladite base (4).

9. Dispositif (2) selon la revendication 8, dans lequel ladite poignée (6) est amovible.

10. Dispositif (2) selon la revendication 7, dans lequel ladite facette de raccord de poignée (4c) et ladite facette abrasive sont disposées sensiblement parallèlement l'une à l'autre sur des côtés opposés de ladite base (4).

11. Dispositif (2) selon la revendication 7, dans lequel ladite base (4) comprend un bord lisse raccordant ladite facette abrasive à ladite facette de raccord de poignée (4c).

12. Dispositif (2) selon la revendication 11, dans lequel ledit bord lisse forme un arc raccordant ladite facette abrasive à ladite facette de raccord de poignée (4c).

13. Dispositif (2) selon la revendication 11, dans lequel ladite facette abrasive est sensiblement circulaire d'un premier diamètre, et ladite facette de raccord de poignée (4c) est sensiblement circulaire d'un second diamètre, et dans lequel ledit second diamètre est supérieur audit premier diamètre.

14. Dispositif (2) selon la revendication 1, dans lequel la surface abrasive (5) est un ensemble comprenant une pluralité de microprotubérances (14).

15. Dispositif (2) selon la revendication 14, dans lequel la substance est revêtue sur les microprotubérances (14).

16. Dispositif (2) selon la revendication 14, dans lequel lesdites microprotubérances (14) sont des microprotubérances tronconiques ou pyramidales coniques (14) comprenant au moins un bord de raclage (20, 22) et faisant saillie de ladite surface abrasive (5).

17. Dispositif (2) selon la revendication 14, dans lequel lesdites microprotubérances (14) sont au moins partiellement revêtues de ladite substance à administrer.

18. Dispositif (2) selon la revendication 14, dans lequel lesdites microprotubérances (14) sont d'une longueur suffisante pour pénétrer dans ou à travers la couche de stratum corneum de ladite peau.

19. Dispositif (2) selon la revendication 14, dans lequel lesdites microprotubérances (14) comprennent au moins deux bords de raclage (20, 22).

20. Dispositif (2) selon la revendication 14, dans lequel lesdites microprotubérances (14) comprennent au moins trois facettes (16) et dans lequel l'intersection de deux quelconques desdites facettes forme un bord de raclage (20, 22).

21. Dispositif (2) selon la revendication 14, dans lequel lesdites microprotubérances (14) comprennent une extrémité plate (18).

22. Dispositif (2) selon la revendication 14, dans lequel lesdites microprotubérances (14) ont des bases de microprotubérances et sont construites et agencées dans un motif de sorte que la distance entre les centres desdites bases de microprotubérances représente au moins deux fois la longueur de ladite microprotubérance.

23. Dispositif (2) selon la revendication 14, dans lequel lesdites microprotubérances (14) ont des bases de microprotubérances et sont construites et agencées dans un motif de sorte que la distance entre les centres desdites bases de microprotubérances représente au moins cinq fois la longueur de ladite microprotubérance.

24. Dispositif (2) selon la revendication 22 ou 23, dans lequel lesdites bases desdites microprotubérances (14) sont espacées pour former des creux entre lesdites microprotubérances (14).

25. Dispositif (2) selon la revendication 14, dans lequel lesdites microprotubérances (14) sont agencées dans un motif.

26. Dispositif (2) selon la revendication 25, dans lequel ledit motif est constitué de rangées et de colonnes ou dans lequel ledit motif est un motif uniforme, circulaire ou aléatoire.

27. Dispositif (2) selon la revendication 14, dans lequel la longueur desdites microprotubérances (14) est supérieure à la profondeur à laquelle elles pénètrent dans ladite peau.

28. Dispositif (2) selon la revendication 14, dans lequel la longueur desdites microprotubérances (14) est
(i) de 5 à 500 microns,
(ii) de 30 à 300 microns,
(iii) de 75 à 250 microns, ou
(iv) de 180 à 220 microns.

29. Dispositif (2) selon la revendication 1, dans lequel ladite surface abrasive (5) est une matière plastique ou du silicium.

30. Dispositif (2) selon la revendication 1, dans lequel ladite substance est une substance bioactive.

31. Dispositif (2) selon la revendication 30, dans lequel ladite substance bioactive est
(i) un médicament,
(ii) un vaccin,
(iii) un allergène, ou
(iv) un agent thérapeutique génique.

32. Dispositif (2) selon la revendication 31 (ii), dans lequel ledit vaccin comprend
(i) un virus ou vecteur viral atténué, vivant,
(ii) un virus inactivé ou tué,
(iii) une bactérie inactivée ou tuée,
(iv) un acide nucléique,
(v) un acide nucléique et une protéine ou un peptide codé par ledit acide nucléique,
(vi) un virus ou une bactérie non atténué vivant,
(vii) un polysaccharide ou un conjugué de polysaccharide, ou
(viii) une protéine ou un peptide.

33. Dispositif (2) selon la revendication 31 (ii) ou 32 (viii), dans lequel ledit vaccin comprend en outre un adjuvant.

34. Dispositif (2) selon l'une quelconque des revendications précédentes, dans lequel, en utilisation, le liquide reconstituant peut être libéré pour dissoudre la substance lorsque le dispositif est appliqué sur la peau pour abrasion.

35. Dispositif (2) selon la revendication 5 ou 6, dans lequel la base (4) est configurée avec une couronne de type champignon (4b) qui se courbe vers le haut et est tronquée au niveau du sommet.

36. Dispositif (2) selon l'une quelconque des revendications précédentes, dans lequel le dispositif comprend en outre un moyen pour réguler la vitesse d'administration de la substance ou du liquide reconstituant.

37. Nécessaire comprenant au moins un dispositif (2) selon la revendication 1.
